# EUROPEAN PATENT SPECIFICATION

(11) **EP 1 920 064 B1**
(45) Date of publication and mention of the grant of the patent: **24.12.2014**
(21) Application number: 06786694.7
(22) Date of filing: 07.07.2006
(51) Int. Cl.: C12Q 1/68

(54) **COMPOSITIONS AND METHODS FOR INCREASING AMPLIFICATION EFFICIENCY**
ZUSAMMENSETZUNGEN UND VERFAHREN ZUR ERHÖHUNG DER AMPLIFIKATIONSEFFIZIENZ
COMPOSITIONS ET METHODES AMELIORANT L'EFFICACITE DE L'AMPLIFICATION

(30) Priority: 07.11.2005 US 733785 P; 12.04.2006 US 744703 P; 07.07.2005 US 696784 P
(43) Date of publication of application: 14.05.2008
(73) Proprietor: Quanta Biosciences, Inc., Gaithersburg, MD 20877 (US)
(72) Inventor: RASHTCHIAN, Ayoub, Gaithersburg, MD 20882 (US); SCHUSTER, David, M., Poolesville, MD 20837 (US)
(74) Representative: Bohmann, Armin K.
(86) International application number: PCT/US2006/026627
(87) International publication number: WO 2007/008728

(56) References cited:
- US-A- 5 418 149
- US-A1- 2004 115 639
- US-A1- 2005 089 922
- US-A1- 2007 009 922
- US-B1- 6 482 590
- US-B1- 6 627 424
- KELLOGG ET AL: "TaqStart Antibody", BIOTECHNIQUES,, vol. 16, no. 6, 1 January 1994 (1994-01-01), pages 1134-1137, XP002103159, ISSN: 0736-6205
- LASKEN ROGER S ET AL: "Archaebacterial DNA polymerases tightly bind uracil-containing DNA", JOURNAL OF BIOLOGICAL CHEMISTRY, THE AMERICAN SOCIETY OF BIOLOGICAL CHEMISTS, INC., BALTIMORE, MD, US, vol. 271, no. 30, 1 January 1996 (1996-01-01), pages 17692-17696, XP002152082, ISSN: 0021-9258, DOI: DOI:10.1074/JBC.271.30.17692
- KAINZ PETER ET AL: "Specificity-enhanced hot-start PCR: Addition of double-stranded DNA fragments adapted to the annealing temperature", BIOTECHNIQUES,, vol. 28, no. 2, 1 February 2000 (2000-02-01), pages 278-282, XP002202181, ISSN: 0736-6205
- DANG C ET AL: "OLIGONUCLEOTIDE INHIBITORS OF TAQ DNA POLYMERASE FACILITATE DETECTION OF LOW COPY NUMBER TARGETS BY PCR", JOURNAL OF MOLECULAR BIOLOGY, LONDON, GB, vol. 264, no. 2, 29 November 1996 (1996-11-29), pages 268-278, XP000852828, ISSN: 0022-2836, DOI: DOI:10.1006/JMBI.1996.0640

## Description

### FIELD OF THE INVENTION

The present invention is related to a method of restoring the polymerase activity of a reversibly inhibited DNA polymerase enzyme, and to a reversibly inactivated DNA polymerase composition.

### BACKGROUND

Technologies for detecting minute quantities of nucleic acids have advanced rapidly over the last two decades, including the development of highly sophisticated methods using various enzymes for *in vitro* amplification of nucleic acids. Assays capable of detecting the presence of a particular nucleic acid molecule in a sample are of substantial importance in forensics, medicine, epidemiology and public health, and in the prediction and diagnosis of disease. Such assays can be used, for example, to identify the causal agent of an infectious disease, to predict the likelihood that an individual will suffer from a genetic disease, and to detect the presence of contaminants in drinking water or milk, and to identify tissue samples.

As an example of amplification, the polymerase chain reaction (PCR) allows detection of very small concentrations of a targeted nucleic acid. Briefly, PCR involves hybridizing primers to the strands of a targeted nucleic acid, also called "templates," in the presence of a polymerization agent, for example a DNA polymerase, and deoxyribonucleoside triphosphates, under the appropriate temperature and buffer conditions. This results in formation of primer extension products along the templates, the products having added thereto nucleotides that are complementary to the templates. Once the primer extension products are denatured, one copy of the template has been prepared, and the cycle of priming, extending and denaturation can be carried out as many times as desired, yielding an exponential increase in the amount of nucleic acid that has the same sequence as the target nucleic acid. In effect, the target nucleic acid is duplicated or amplified many times in order to increase detection of the target sequence. Despite the broad and rapid use of PCR in a variety of biological and diagnostic fields, there are still practical limitations that must be overcome to achieve optimum success of the technology. For example, PCR produces considerable inefficiency in the use of expensive reagents.

Many amplification procedures yield nonspecific side-products. Sometimes, this nonspecificity results from mispriming, which occurs when primers anneal to non-target nucleic acids. For example, in addition to amplified target nucleic acids, PCR also yields primer dimers or oligomers and double-stranded side products containing the sequences of several primer molecules joined end-to-end. These unwanted products may adversely affect accurate and sensitive detection of the target nucleic acid.

The problem caused by unwanted side products is particularly acute when the target nucleic acid is present in very low concentrations, for example, less than about 1000 molecules. A low concentration of target nucleic acid is often seen in early stages of infectious diseases or in very small specimens, such as may be the situation with forensic investigations.

Under ideal PCR conditions, the primers used anneal very specifically to the target nucleic acid only. Target-specific annealing occurs most often when elevated and optimized temperatures are used in the PCR process. However, the reaction mixture may also be held at lower temperatures for certain reasons, for example during manufacture, shipping, or before use by a customer. When the reaction mixtures are held at lower temperatures the primers may undesirably bind non-target nucleic acids. For example, in high-throughput applications involving robotics for reaction assembly, reactions are held at ambient temperatures for extended period of time prior to the start of the amplification process. If this occurs, nonspecific primer extension products and primer dimers can form. These byproducts can be amplified along with the target nucleic acid during PCR. These undesired byproducts may obscure any amplified target nucleic acid (e.g., high background) or prevent accurate and quantitative amplification of targeted nucleic acids. Moreover, because the reaction reagents are utilized to make nonspecific products, less specific product is produced, rendering the entire PCR reaction less sensitive tor detecting target nucleic acid and more costly. In testing for diagnostic purposes this can result in false negative results.

Extensive work has been carried out to isolate and characterize DNA polymerases from many sources that are suitable for use in amplification reactions. Thermostable DNA polymerases have been found to be advantageous in PCR because of their stability at high temperatures, which are required during certain steps of PCR. These thermostable DNA polymerases do not, however, solve the problem of nonspecific amplification or primer dimer formation. These two problems are particularly acute when a thermostable DNA polymerase is used in the PCR reaction mix because thermostable DNA polymerases retain activity even at relatively lower temperatures (e.g. below about 50°C).

Most thermostable DNA polymerases have a low level of polymerization activity at lower temperatures making this a virtually universal problem in PCR. Although the performance of PCR at elevated temperatures has reduced the level of nonspecific annealing of primers to polynucleotide sequences in the reaction mixture, especially at the elevated temperatures required for optimum thermostable polymerase activity, nonspecific primer interactions with polynucleotide sequences, and some level of corresponding primer elongation by the thermostable polymerase, does occurs at lower temperatures. The nonspecific interactions and activity of the thermophilic polymerase tends to occur even at temperatures as low as 25°C -- i.e., during the set-up of the PCR reaction mixture at room temperature, especially when a large number of reactions are prepared simultaneously. For example, the most frequently used thermostable DNA polymerase, *Thermus aquaticus* (Taq) DNA polymerase, which is fully activity at 70°C is still 12-15% active at 30°C. This problem is especially prevalent in PCR applications having a small number of target polynucleotide sequences in a milieu containing an excess of non-target (e.g., nonspecific) DNA and/or RNA. It would be desirable to reduce or eliminate the formation of nonspecific products and primer dimers during PCR amplification. In fact, several approaches have been advanced within the art to minimize these inherent shortcomings in PCR. One common method is to withhold addition of polymerase to reactions until the reaction temperatures have reached 80°C or higher and then individually add the enzyme while reactions are in the thermocycler. This method is referred to as "manual hot start" and works well for processing a small number of samples.

The overall approach to hot start PCR reactions is to physically, chemically or biochemically block the polymerization reaction until the reaction reaches a temperature equal to the optimal annealing temperature of the primers. In this manner the thermostable polymerase is unable to elongate primer-template polynucleotides at temperatures where nonspecific primer template DNA interactions can exist. With regard to physical hot start PCR, the thermostable polymerase, or one of the other critical reaction components (e.g. dNTPs or magnesium ions) is withheld from the reaction until the reaction reaches temperatures in the range of 85°C. to 95°C. This temperature is sufficiently high enough to prevent even partial hybridization of the primers to the template polynucleotide. As such, substantially no nonspecific primer annealing to polynucleotides occurs in the reaction mixture.

A number of physical blocks can be used to partition the reaction in a heat dependent manner, including, a wax barrier or wax beads with embedded reaction components, which melts at around 55°C to 65°C. However, a shortcoming to using these wax barriers or wax beads is that the melted material remains in the reaction for the duration of the PCR, forming a potential inhibitor for some PCR applications as well as being incompatible with some potential downstream applications of the amplified product. In some cases the barrier can be physically removed from the reaction to accommodate later uses, but the removal increases the risk of sample-to-sample contamination and requires time and energy to accomplish.

A second physical hot start PCR technique utilizes a compartmentalized tube in a temperature regulated centrifuge. The components of the PCR reaction are compartmentalized within the tube from a critical component of the PCR reaction, where the components are all brought together by rupturing the compartments of the tube at a certain g-force that corresponds to the specific annealing temperatures of the primer-template polynucleotide. This is accomplished by a dedicated centrifuge that regulates g force with rotor temperature. However, this technique requires expensive equipment-compartmentalized tubes for each PCR reaction and a specialized centrifuge--each factor limiting the number of reactions that can be run at one time and increasing the cost of each reaction.

"Another way to implement hot start PCR is to use a thermostable polymerase that has been reversibly inactivated by a chemical modification, such as AmpliTaq Gold™ DNA polymerase. [Birch et al., 1998, U.S. Pat. No. 5,773,258; Ivanov et al., 2001, U.S. Pat. No. 6,183,998.] These techniques are generally referred to as chemical hot start PCR. In the most common type of chemical hot start PCR, the thermostable polymerase, typically Taq DNA polymerase, has been chemically cross-linked to inactivate the enzyme. The nature of the cross-linkers and the chemical bonds formed in these methods are reversible and the cross-linked thermostable polymerase is reactivated by heating the polymerase prior to the reaction for a predetermined amount of time at 95°C and at a specific pH. [Moretti et al. (1998) Biotechniques 25:716-725.] The optimal pH for the destruction of the cross-links at 95°C is adjusted by using reaction buffers which have a pH of 8.0 at 25°C. However, this buffer pH is suboptimal for the activity of the thermostable polymerase at 65-70°C in the elongation step during PCR. Another major drawback of the technique is that only a fraction of the enzyme is ever reactivated through heating, leaving a substantial part (up to 50%) of the enzyme in a permanently inactive state. Also, the degree of chemical modification is difficult to normalize between various polymerase preparations, and therefore results in batch-to-batch variations of the polymerase activity. This approach has proven to be costly and ineffective for polymerizing longer stretches of target nucleic acid sequence. In addition, the investigator is limited to the use of the chemically-modified polymerase and therefore the reaction conditions required for that chemically-modified polymerase.

A third way of implementing hot start PCR is by combining a monoclonal antibody, which is specific to the thermostable polymerase, with the thermostable polymerase before addition to the PCR reaction. This type of hot start PCR can be referred to as hot start PCR by affinity ligand blocking. The antibody binds to the thermostable polymerase at lower temperatures and blocks activity, but is denatured at higher temperatures, thus rendering the polymerase active. [Scalice et al. (1994) J. Immunol. Methods, 172:147-163; Scalice et al. U.S. Pat. No. 5,338,671; Sharkey et al. (1994) Bio/Technology, 12:506-509; Kellogg et al. (1994) Biotechniques, 16: 11341137.]. A shortcoming of the affinity ligand blocking hot start PCR is that due to the high specificity of monoclonal antibodies they cannot be used with other DNA polymerases. A more general method that can be applied to a variety of DNA polymerases would be more desirable.

An alternative ligand blocking hot start PCR technique has been developed based on aptamers. Aptamers are single-stranded oligonucleotides possessing a DNA sequence with high binding affinity for the active center of selected thermophilic DNA polymerases. [Gold et al. 2000, U.S. Pat. No. 6,020,130; Jayasena et al. 2001, U.S. Pat. No. 6,183,967]. The single-stranded oligonucleotides bind to the thermophilic polymerase at lower temperatures and are released at higher temperatures. However, as with to the monoclonal antibody technique, aptamers are also polymerase specific and are not generally applicable to all polymerases. In contrast to all previously discussed techniques for hot start PCR, competitive oligonucleotide aptamers remain active throughout the PCR and not only prior to the first PCR cycle. In practical terms, however, these nucleic acid competitors have undesirable effects in reducing sensitivity of PCR because they remain active in the PCR reaction during polymerization and they compete with amplification of desired sequences.

More recently, another hot start PCR approach has been developed that involves using a genetically engineered Taq DNA polymerase that contains mutations, which render the enzyme inactive below 35°C. [Barnes et al.; 2001; U.S. Pat. No. 6,214,557.] However, this N-terminally truncated form of Taq DNA polymerase has about a five-fold lower processivity (i.e., the number of nucleotides polymerized by a DNA polymerase during a single association-dissociation cycle with the primer-template) than wild type Taq DNA polymerase, thereby requiring a 5-10 fold activity excess of the enzyme in each PCR reaction as compared to the wild type Taq DNA polymerase. As a result, this technique is limited to the amplification of short target sequences (e.g. target sequences <1 kb). It remains to be determined whether the mutations causing inactivation of the truncated Taq DNA polymerase at lower temperatures, would have the same effect when engineered into a full-length Taq DNA polymerase.

It has also been found that homopolymeric stretches of DNA possess inhibitory activity on several types of eucaryotic DNA polymerases ((Shimada et al., 1978 Nucl.Acids Res., Vol 5, Issue 9 3427-3438, 1978; Oguro et al. Nucl.Acids Res. 1979 October 10; 7(3): 727-734.). Recently, Kainz described the inhibition ot PCR by the addition of nonspecific double-stranded DNA from *Escherichia coli* phage lambda. (Kainz et al., (2000) Biochim Biophys Acta, 28(2):278-82.) Kainz and coworkers proposed a mechanism for DNA inhibition where an excess of nonspecific double-stranded ("ds") DNA binds up the available active Taq DNA polymerase and argued that this feature of Taq DNA polymerase provides the reason for saturation of the PCR amplification reaction during late cycles. In effect, all available free Taq DNA polymerase is bound up by the accumulated ds PCR product. This proposed effect was employed to inhibit Taq DNA polymerase at ambient temperatures with an excess of small ds oligonucleotides. [Kainz et al., (2000) Biotechniques, 15:1494(1-2):23-7.] While these methods are somewhat effective to inhibit polymerase activity prior to PCR, the ds oligonucleotides remain present throughout PCR process and result in reduced PCR efficiency. The reduced PCR efficiency is particularly problematic for quantitative PCR.

The inhibitory effect of natural and synthetic polyanions [Holler *et al.,* 1992, Holler *et al.,* Shimada *et al.,* 1978], in particular of sulfated polysaccharides [Hitzeman *et al.,* 1978], on various DNA and RNA polymerases [Ferencz *et al.,* 1975] has been well known for many years. Acid polyanionic polysaccharides have been characterized as the major PCR inhibitor in plant DNA isolations [Demeke *et al.,* 1992], whereas sulfated polysaccharides, such as dextran sulfate and heparin were identified as potent PCR inhibitors contaminating DNA preparations from blood cells [Al-Soud *et al.,* 2001]. Sulfated polysaccharides in particular show a broad spectrum of inhibition against a variety of DNA-modifying enzymes including polynucleotide kinase [Wu *et al.,* 1971], restriction endonucleases [Do *et al.,* 1991] and retroviral reverse transcriptases [Moelling et al., 1989]. Although the inhibitory effect of polyanions and sulfated polysaccharides in particular has been studied for many years, the exact mechanism is not known [Furukawa *et al.,* 1983]. Also the factors determining the degree of inhibition other than the concentration of the polyanion have not yet been studied systematically. It has been suggested that anionic polysaccharides are competitive inhibitors of DNA- and RNA modifying enzymes competing with the substrate nucleic acids for binding the enzyme. The chemical structure of anionic acidic polysaccharides resembles the polypentose phosphate structure of the backbone of nucleic acids. Based on this principle DNA and RNA polymerases, DNA binding enzymes, and in particular, Taq DNA polymerase are purified by affinity chromatography on heparin sepharose. Recently, several single amino acid substitutions both on the polymerase and N-terminal exonuclease domain of Taq DNA polymerase have been found to drastically reduce the susceptibility of Taq DNA polymerase for inhibition by heparin (Ghadessy *et al.,* 2001). This represents the direct experimental indication that the inhibitive effect of heparin is related to binding of this sulfated polysaccharide to certain sites of the DNA polymerase molecule.

Lasken *et al.* have demonstrated that uracil containing DNA can bind archaebacterial polymerases.[Lasken et al., J. Biol. Chem, Vol. 271: 17692-17696, 1996.]. This inhibition was reported as as a problem for use of archaebacterial polymerases when dU residues are present in DNA. Others also identified this inhibition as a problem for efficient PCR (Hogrefe, et al. Proc. Nat'l Acad. Sci USA Vol 99:596 (2002). These investigators feared that presence of contaminating dUTP in the nucleotide mix or conversion of dCTP to dUTP (during high temperature steps of PCR) and subsequent incorporation of dUTP into DNA could inhibit Pfu polymerase and reduce PCR efficiency. Consequently they identified a thermostable dUTPase that could be used to overcome this problem.

On the other hand, Gelfand et al. (US patent 5418149) found dUTP to be useful in PCR and included dUTP in the nucleotide triphosphate mix. It was found that the presence of dUTP and use of a thermolabile UDG removed some of the mispriming products that had been generated by incorporation of dUTP and improved PCR specificity. Unlike other methods described above, this method did not inhibit polymerase activity, rather it degraded some of the non-specifically generated DNA fragments (those generated by incorporation of dU), thereby improving PCR specificity.

Kainz P et al. (Biotechniques, vol. 28, no. 2, 1 February 2000, pp. 278-282) describe specificity-enhanced hot-start PCR in which double-stranded DNA fragments adapted to the annealing temperature are used.

Dang C et al. (Journal of Molecular Biology, vol. 264. No. 2. 29 November 1996, pp. 268-278) describe the use of oligonucleotide inhibitors of *Taq* DNA polymerase for facilitating detection of low copy number targets by PCR.

It is apparent, therefore, that present methods of nucleic amplification suffer from a variety of drawbacks and disadvantages, and that improved amplification methods are greatly to be desired. In particular, new and improved methods for "hot-start" nucleic acid amplification would be highly desirable.

### SUMMARY OF THE INVENTION

It is therefore an object of the present invention to provide methods for improving the efficiency of nucleic acid amplification, in particular to provide methods for reversibly inhibiting DNA polymerase activity at temperatures that lead to formation of unwanted products during amplification.

It is also an object of the present invention to provide compositions, including DNA polymerase enzymes that are reversibly inhibited by the presence of a binding partner, and enzymes that modify the binding partnercan products, including genomic DNA collections and seed assemblages of particular constituency, that are particularly adapted to implementing such a method.

The problem underlying the present invention is solved by the subject matter of the attached independent claims. Preferred embodiments may be taken from the attached dependent claims.

More specifically, the problem underlying the present invention is solved in a first aspect by a method of restoring the polymerase activity of a reversibly inhibited DNA polymerase enzyme, comprising:
contacting said polymerase enzyme with a second enzyme,
wherein said second enzyme modifies a nucleic acid binding partner that is bound to said polymerase enzyme,
wherein the polymerase activity of said polymerase enzyme is substantially inhibited by said binding partner,
and wherein modification of said binding partner by said second enzyme restores polymerase activity of said polymerase enzyme.

In an embodiment of the first aspect, said DNA polymerase is a thermostable DNA polymerase.

In an embodiment of the first aspect, said binding partner is non-covalently bound to said polymerase.

In an embodiment of the first aspect, said binding partner is a single stranded nucleic acid.

In an embodiment of the first aspect, said binding partner is a double stranded nucleic acid.

In an embodiment of the first aspect, said binding partner is covalently bound to said polymerase.

In an embodiment of the first aspect, said nucleic acid comprises a plurality of deoxyuridine residues and wherein said second enzyme has DNA glycosylase activity.

In an embodiment of the first aspect, said second enzyme has DNA glycosylase activity.

In an embodiment of the first aspect, said nucleic acid comprises at least one deoxyuridine residue, and wherein said second enzyme has UDG activity.

In an embodiment of the first aspect, said nucleic acid is covalently bound to said polymerase by a covalent linkage that is heat-labile or hydrolytically labile during PCR temperature cycling.

In an embodiment of the first aspect, said nucleic acid is covalently bound to said polymerase by a covalent linkage that is not heat-labile or hydrolytically labile during PCR temperature cycling.

In an embodiment of the first aspect, said covalent linkage comprises an amide linkage, a urethane linkage, or a urea linkage.

In an embodiment of the first aspect, said linkage comprises an amide linkage.

In an embodiment of the first aspect, said nucleic acid comprises an RNA oligonucleotide, and said second enzyme has RNAse activity.

In an embodiment of the first aspect, nucleic acid is a double stranded nucleic acid comprising a DNA/RNA duplex and wherein said second enzyme has RNAseH activity.

In an embodiment of the first aspect, said nucleic acid comprises a DNA/DNA duplex and wherein said second enzyme has restriction endonuclease activity that cleaves said duplex.

In an embodiment of the first aspect, said second enzyme cuts at a 6-8 base pair recognition site.

In an embodiment of the first aspect, contacting said polymerase enzyme with a second enzyme, comprises:
contacting said polymerase enzyme with a second enzyme and an antibody that binds to said second enzyme and inhibits the activity of said enzyme at ambient temperature,
and wherein under PCR temperature cycling conditions the inhibition of activity of said second enzyme by said antibody is reduced or eliminated and modification of said binding partner by said second enzyme restores polymerase activity of said polymerase enzyme.

In an embodiment of the first aspect, said DNA polymerase is a thermostable DNA polymerase.

In an embodiment of the first aspect, said binding partner is non-covalently bound to said polymerase.

In an embodiment of the first aspect, said binding partner is covalently bound to said polymerase.

In an embodiment of the first aspect, said nucleic acid comprises a plurality of deoxyuridine residues and wherein said second enzyme has DNA glycosylase activity.

In an embodiment of the first aspect, said second enzyme has DNA glycosylase activity.

In an embodiment of the first aspect, said nucleic acid comprises at least one deoxyuridine residue, and wherein said second enzyme has UDG activity.

In an embodiment of the first aspect, said nucleic acid comprises an RNA oligonucleotide, and said second enzyme has RNAse activity.

In an embodiment of the first aspect, said nucleic acid is a double stranded nucleic acid comprising a DNA/RNA duplex and wherein said second enzyme has RNAseH activity.

In an embodiment of the first aspect, said polymerase is an archaebactaerial polymerase.

In an embodiment of the first aspect, said polymerase is Pfu.

In an embodiment of the first aspect, said polymerase is a Eubacterial DNA polymerase.

In an embodiment of the first aspect, said polymerase is Taq DNA polymerase.

In an embodiment of the first aspect, said binding partner is a single stranded nucleic acid.

In an embodiment of the first aspect, said binding partner is a double stranded nucleic acid.

In an embodiment of the first aspect, said nucleic acid comprises at least one modified deoxyribonucleotide.

In an embodiment of the first aspect, said nucleic acid comprises at least one deoxyuridine residue modifiable by an enzyme having UDG activity.

In an embodiment of the first aspect, said nucleic acid comprises a DNA/DNA duplex and wherein said second enzyme has restriction endonuclease activity that cleaves said duplex.

In an embodiment of the first aspect, said second enzyme cuts at a 6-8 base pair recognition site.

More specifically, the problem underlying the present invention is solved in a second aspect by a reversibly inactivated DNA polymerase composition comprising a reversibly inactivated DNA polymerase, a nucleic acid binding partner bound to the DNA polymerase, and a second enzyme, wherein said DNA polymerase is active in the absence of said binding partner, and substantially inactive in the presence of said binding partner, and
wherein said binding partner is modifiable by the second enzyme, whereby modification of said binding partner by said second enzyme restores polymerase activity to said polymerase enzyme.

In an embodiment of the second aspect, said polymerase is an archaebactaerial polymerase.

In an embodiment of the second aspect, said polymerase is Pfu.

In an embodiment of the second aspect, said polymerase is a Eubacterial DNA polymerase.

In an embodiment of the second aspect, said polymerase is Taq DNA polymerase.

In an embodiment of the second aspect, said DNA polymerase is a thermostable DNA polymerase.

In an embodiment of the second aspect, said binding partner is non-covalently bound to said polymerase.

In an embodiment of the second aspect, said nucleic acid comprises at least one modified deoxyribonucleotide.

In an embodiment of the second aspect, said nucleic acid comprises at least one deoxyuridine residue modifiable by an enzyme having UDG activity.

In an embodiment of the second aspect, said binding partner is covalently bound to said polymerase.

In an embodiment of the second aspect, said nucleic acid comprises at least one deoxyuridine residue modifiable by an enzyme having UDG activity.

In an embodiment of the second aspect, said nucleic acid is covalently bound to said polymerase by a covalent linkage that is not heat-labile or hydrolytically labile during PCR temperature cycling.

In an embodiment of the second aspect, said covalent linkage comprises an amide linkage, a urethane linkage, or a urea linkage.

In an embodiment of the second aspect, said linkage comprises an amide linkage.

In an embodiment of the second aspect, said nucleic acid comprises an RNA oligonucleotide.

In an embodiment of the second aspect, said nucleic acid is a double stranded nucleic acid comprising a DNA/RNA duplex that is recognized by an enzyme having RNAseH activity.

In an embodiment of the second aspect, said nucleic acid comprises a DNA/DNA duplex containing a recognition site recognized by a restriction endonuclease.

In an embodiment of the second aspect, said recognition site is 6-8 bases long.

In accomplishing these objects, there has also been disclosed a method of reversibly inhibiting the polymerase activity of a DNA polymerase enzyme by contacting the polymerase enzyme with a second enzyme that modifies a binding partner that is bound to the polymerase enzyme and substantially inhibits the polymerase activity of the polymerase enzyme when bound, where the modification of the binding partner by the second enzyme restores polymerase activity of the polymerase enzyme. The DNA polymerase is, *e.g.,* a thermostable DNA polymerase. The binding partner is non-covalently bound to the polymerase. Alternatively, the binding partner is covalently bound to the polymerase. In embodiments of the invention the binding partner is a nucleic acid. The nucleic acid is a single stranded nucleic acid. Alternatively, the nucleic acid is a double stranded nucleic acid. In other embodiments the second enzyme has DNA glycosylase activity. For example, the nucleic acid contains a plurality of deoxyuridine residues and the second enzyme has DNA glycosylase activity. In still other embodiments the nucleic acid contains at least one deoxyuridine residue and the second enzyme has UDG activity. Alternatively, the nucleic acid comprises an RNA oligonucleotide, and the second enzyme has RNAse activity. For example, the nucleic acid is a double stranded nucleic acid containing a DNA/RNA duplex and the second enzyme has RNAseH activity. In other embodiments, the nucleic acid contains a DNA/DNA duplex and the second enzyme has restriction endonuclease activity that cleaves the duplex, such as where the second enzyme cuts at a 6-8 base pair recognition site.

In other embodiments, the binding partner contains a peptide and the second enzyme has a protease activity that cleaves the peptide. The binding partner contains a peptide having a covalent modification and the second enzyme has activity that cleaves the covalent modification. The covalent modification includes, e.g., at least one phosphate group linked to a serine, threonine or tyrosine residue of the peptide and the second enzyme has a suitable protein phosphatase activity.

In still other embodiments, the binding partner contains a lipid and the second enzyme has lipase activity.

In some embodiments, the nucleic acid is covalently bound to the polymerase by a covalent linkage that is heat-labile or hydrolytically labile during PCR temperature cycling. Alternatively, the nucleic acid is covalently bound to the polymerase by a covalent linkage that is not heat-labile or hydrolytically labile during PCR temperature cycling. The covalent linkage contains, e.g., an amide linkage, a urethane linkage, or a urea linkage.

In another aspect, the invention provides a reversibly inactivated DNA polymerase composition as defined in the claims that contains a binding partner bound to a DNA polymerase, where the DNA polymerase is active in the absence of the binding partner and substantially inactive in the presence of the binding partner, and where the binding partner is modifiable by a second enzyme, such that a modification of the binding partner by the second enzyme restores polymerase activity to the polymerase enzyme. The DNA polymerase is a thermostable DNA polymerase. The binding partner is non-covalently bound to the polymerase. Alternatively, the binding partner is covalently bound to the polymerase. The polymerase is an archaebactaerial polymerase, Pfu polymerase, a Eubacterial DNA polymerase, or Taq DNA polymerase.

The binding partner is, *e.g.,* a nucleic acid. In embodiments, the nucleic acid contains at least one deoxyuridine residue modifiable by an enzyme having UDG activity. In certain embodiments, the nucleic acid is covalently bound to the polymerase by a covalent linkage that is not heat-labile or hydrolytically labile during PCR temperature cycling. The covalent linkage contains, for example, an amide linkage, a urethane linkage, or a urea linkage. In other embodiments, the nucleic acid contains at least one modified deoxyribonucleotide, such as a deoxyuridine residue modifiable by an enzyme having UDG activity.

In an embodiment, the nucleic acid includes an RNA oligonucleotide, or a double stranded nucleic acid containing a DNA/RNA duplex that is recognized by an enzyme having RNAseH activity. Alternatively, the nucleic acid contains a DNA/DNA duplex containing a recognition site recognized by a restriction endonuclease. Optionally, the recognition sites is 6-8 bases long.

In other embodiments, the binding partner contains a peptide including a protease cleavage site, or a peptide containing a covalent modification. The covalent modification includes at least one phosphate group linked to a serine, threonine or tyrosine residue of the peptide, where the phosphate is cleavable by an enzyme having protein phosphatase activity.

In other embodiments, the binding partner includes a lipid that is cleavable by an enzyme having lipase activity.

In a further aspect, the invention provides a composition containing a modified DNA polymerase and a binding moiety. The DNA polymerase is modified by covalent attachment of a binding target that is not an amino acid sequence of the non-modified DNA polymerase, where the modified polymerase is active in the absence of temperature sensitive binding of the binding moiety to the binding target and substantially inactive when the binding moiety is bound to the binding target. In one embodiment, the binding target is biotin and the second moiety is either streptavidin or an antibody that binds to biotin. In another embodiment, the binding target contains an amino acid sequence fused to the amino acid sequence of the non-modified polymerase and the binding moiety is an antibody that binds the binding target. In a further embodiment, the binding target is a nucleic acid and the binding moiety is a protein that binds the binding target, such as an antibody or a transcription factor.

Also disclosed is a method of reversibly inhibiting the polymerase activity of a DNA polymerase enzyme by contacting the polymerase enzyme with a second enzyme and an antibody that binds to the second enzyme that modifies a binding partner that is bound to the polymerase enzyme and inhibits the activity of the enzyme at ambient temperature. The polymerase activity of the polymerase enzyme is substantially inhibited by the binding partner. Under PCR temperature cycling conditions the inhibition of activity of the second enzyme by the antibody is reduced or eliminated and modification of the binding partner by the second enzyme restores polymerase activity of the polymerase enzyme. The DNA polymerase is a thermostable DNA polymerase. The binding partner is non-covalently bound to the polymerase. Alternatively, the binding partner is covalently bound to the polymerase.

In embodiments, the binding partner is a nucleic acid. The nucleic acid is a single stranded nucleic acid or a double stranded nucleic acid. Optionally, the nucleic acid contains at least one modified deoxyribonucleotide. In some embodiments, the nucleic acid includes at least one deoxyuridine residue modifiable by an enzyme having UDG activity. In other embodiments, the nucleic acid contains a plurality of deoxyuridine residues and the second enzyme has DNA glycosylase activity. The second enzyme has DNA glycosylase activity, such as UDG activity when the nucleic acid contains at least one deoxyuridine residue. In another embodiment, the nucleic acid includes an RNA oligonucleotide and the second enzyme has RNAse activity. For example, the nucleic acid is a double stranded nucleic acid containing a DNA/RNA duplex and the second enzyme has RNAseH activity. In other embodiments, the nucleic acid contains a DNA/DNA duplex and the second enzyme has restriction endonuclease activity that cleaves the duplex, such as at a 6-8 base pair recognition site. The polymerase is an archaebactaerial polymerase, Pfu polymerase, a Eubacterial DNA polymerase, or Taq DNA polymerase.

In other embodiments, the binding partner includes a peptide and the second enzyme has protease activity that cleaves the peptide. The binding partner contains a peptide having a covalent modification and the second enzyme has activity that cleaves the covalent modification. The covalent modification can include at least one phosphate group linked to a serine, threonine or tyrosine residue of the peptide and the second enzyme has protein phosphatase activity.

In other embodiments, the binding partner includes a lipid and the second enzyme has lipase activity.

In a further aspect, the invention provides a composition that contains a modified DNA polymerase and a binding target, a second enzyme and an antibody. The DNA polymerase is modified by covalent attachment of the binding target such that the modified polymerase is active in the absence of binding of the binding target to the polymerase and substantially inactive when bound to the binding target. Generally, the binding target is not an amino acid sequence of the non-modified DNA polymerase, and binding of the binding moiety to the binding target is temperature sensitive. The second enzyme is capable of modifying the binding target such that the binding target no longer binds to the polymerase. The antibody binds to the second enzyme at ambient temperature in a manner that substantially inhibits activity of the enzyme, while under conditions of PCR temperature cycling the inhibition of activity of the second enzyme by the antibody is reduced or eliminated and modification of the binding partner by the second enzyme restores polymerase activity of the polymerase enzyme. The DNA polymerase is a thermostable DNA polymerase. For example, the polymerase is an archaebactaerial polymerase, Pfu polymerase, a Eubacterial DNA polymerase, or Taq DNA polymerase. The binding partner is a nucleic acid, such as a single stranded nucleic acid or a double stranded nucleic acid. For example, the nucleic acid has at least one deoxyuridine residue modifiable by an enzyme having UDG activity and the second enzyme has UDG activity. In another embodiment, the nucleic acid includes an RNA oligonucleotide and the second enzyme has RNAse activity. In a further embodiment, the nucleic acid is a double stranded nucleic acid containing a DNA/RNA duplex that is recognized by an enzyme having RNAseH activity, and the second enzyme has RNAseH activity. Alternatively, the nucleic acid contains a DNA/DNA duplex that includes a recognition site recognized by a restriction endonuclease and the second enzyme has restriction endonuclease activity. The nucleic acid is covalently bound to the polymerase by a covalent linkage that is not heat-labile or hydrolytically labile during PCR temperature cycling. The covalent linkage includes, *e.g*., an amide linkage, a urethane linkage, or a urea linkage.

Other objects, features and advantages of the present invention will become apparent from the following detailed description. It should be understood, however, that the detailed description and the specific examples, while indicating preferred embodiments of the invention, are given by way of illustration only.

### BRIEF DESCRIPTION OF THE DRAWINGS

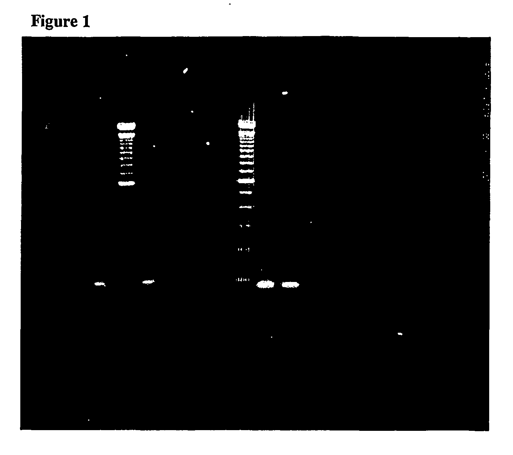

**Figure 1** is a photographic image of a stained agarose gel electrophoresis of PCR amplified fragments using Pfu DNA polymerase. PCR amplifications were performed as described in Example 1. Lane 1-4 show amplification using primer set 20 and varying amounts of dU-containing inhibitor. **Lane 1:** No inhibitor; **Lane 2:** 80 ng; **Lane 3:** 160 ng; **Lane 4:** 320 ng. Lane 5-8 show amplification using primer set 21 and varying amounts of dU-containing inhibitor. **Lane 5:** No inhibitor; Lane 6: 80 ng; **Lane 7:** 160 ng; **Lane 8:** 320 ng. Molecular weight standards are the 100- base pair ladder.

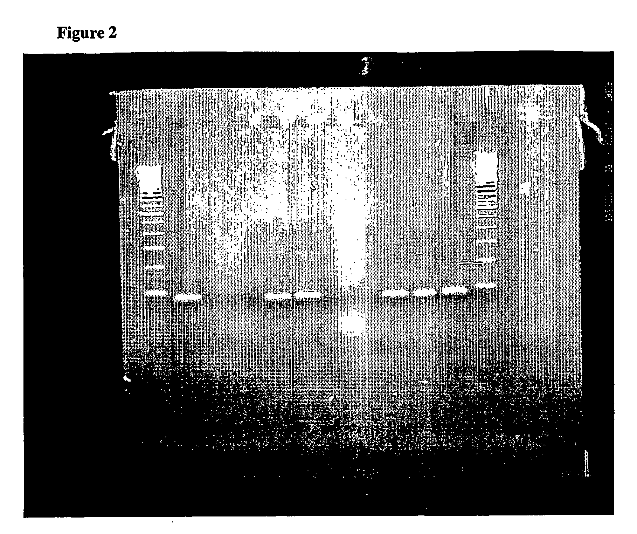

**Figure 2** is a photographic image of a stained agarose gel electrophoresis of PCR amplified fragments using Pfu DNA polymerase. PCR amplifications were performed as described in example 2. Two different dU-containing inhibitors were tested and inhibition was reversed by using UDG. **Lane 1:** No dU inhibitor; **Lane 2:** Inhibitor E-up 100nM; **Lane 3:** Inhibitor E-up 200nM; **Lane 4:** Inhibitor E-up 100nM and 1 unit of *E*. *coli* UDG; **Lane 5:** Inhibitor E-up 200nM and 1 unit of *E. coli* UDG; **Lane 6:** Inhibitor T 100nM; **Lane 7:** Inhibitor T 200nM; **Lane 8:** Inhibitor T 100nM and 1 unit of *E*. *coli* UDG; **Lane 9:** Inhibitor T 200nM and 1 unit of *E. coli* UDG; **Lane 10:** Inhibitor T 100nM and 1 unit of Tth UDG. Inhibitor E-up has the following sequence: 5' agcggauaacaauaucaca 3' (3' end of this oligonucleotide is blocked); Inhibitor T has the following sequence: 5' tgcgaauuccagccucuccagaaaggccc3' (3' end is not blocked).

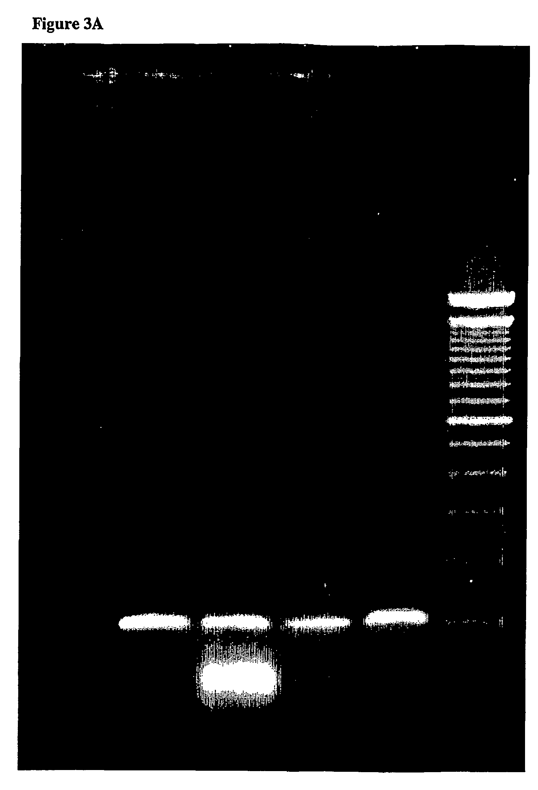

**Figure 3a** is a photographic image of a stained agarose gelAgarose gel electrophoresis of PCR amplified TRRAP gene fragment using various methods of PCR. As described in Example 3 TRRAP gene was amplified under different conditions which included hot start or non hot start conditions. **Lane 1:** Taq DNA polymerase without hot start; **Lane 2:** antibody mediated hot-start using iTaq DNA polymerase; **Lane 3:** Taq DNA polymerase with AR3 polymerase inhibitor; **Lane 4:** Taq DNA polymerase with AR3 polymerase inhibitor and UDG from *Thermatoga maritima*; **Lane 5:** Taq DNA polymerase with AR3 polymerase inhibitor and UDG from *Thermus thermophilus*; **Lane 6:** 100 base pair molecular weight standard.

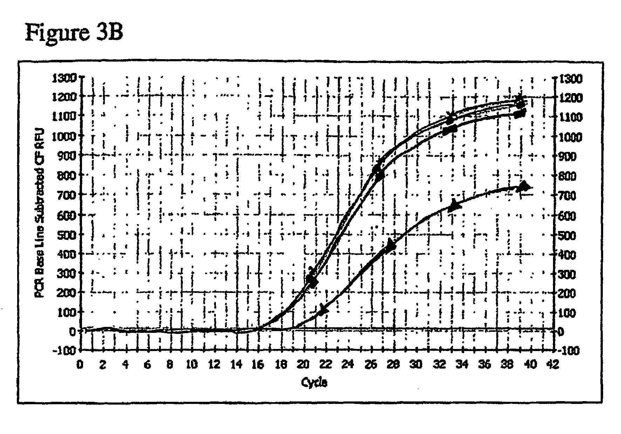

**Figure 3b** is a line graph showing the results of SYBR Green real time PCR: As described in Example 3, the TTR gene fragment was amplified using a number of different conditions. The PCR reactions contained SYBR green for real time detection and were monitored using IQ real time thermocycler (BioRad). The amplification profiles are as follows: ◢ :Taq DNA polymerase; ● : Taq DNA polymerase with AR3 Inhibitor; ■ : Taq DNA polymerase with AR3 Inhibitor and 0.5 units Tth Uracil DNA glycosylase; **X** : iTaq DNA polymerase.

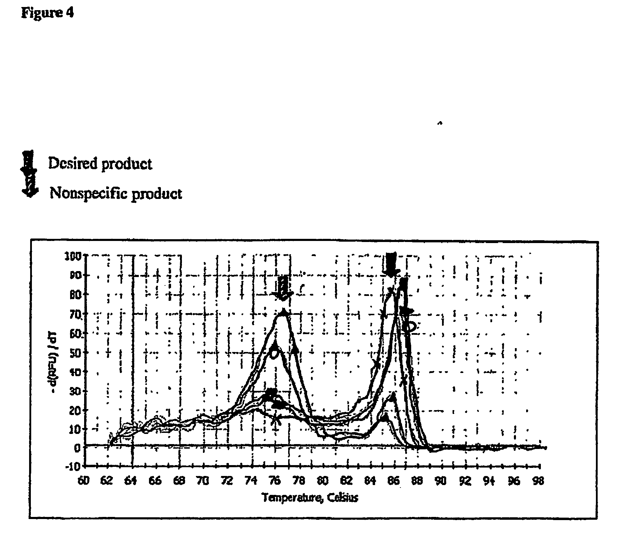

**Figure 4** is a line graph showing the results of melting curve analysis of amplification products. The 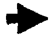 arrow shows the non-specific primer-dimer product. The ⇒ arrow shows the expected amplicon generated by amplification of the correct genomic sequence. The enzymes used for amplification and their method of preparation are shown in Table 3.

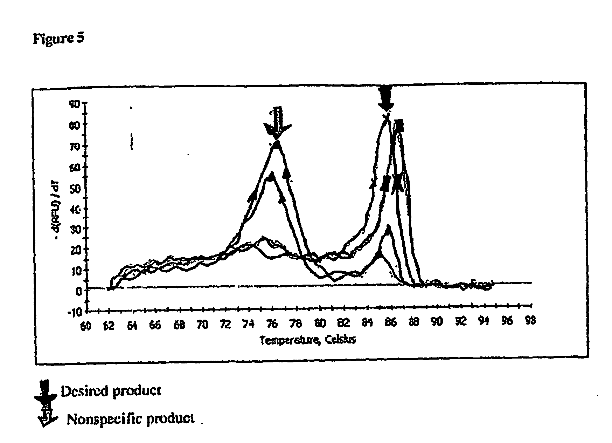

**Figure 5** is a line graph showing the results of melting curve analysis of amplification products. The 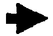 arrow shows the non-specific primer-dimer product The ⇒ arrow shows the expected amplicon generated by amplification of the correct genomic sequence. The enzymes used for amplification and their method of preparation are shown in Table 3.

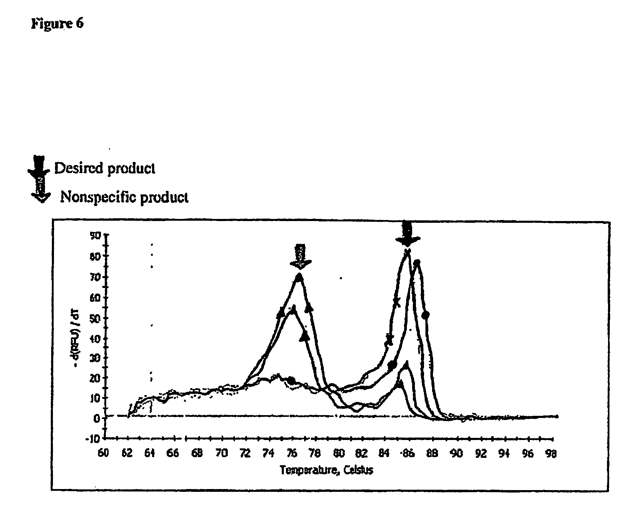

**Figure 6** is a line graph showing the results of melting curve analysis of amplification products. The 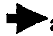 arrow shows the non-specific primer-dimer product. The ⇒ arrow shows the expected amplicon generated by amplification of the correct genomic sequence. The enzymes used for amplification and their method of preparation are shown in Table 3.

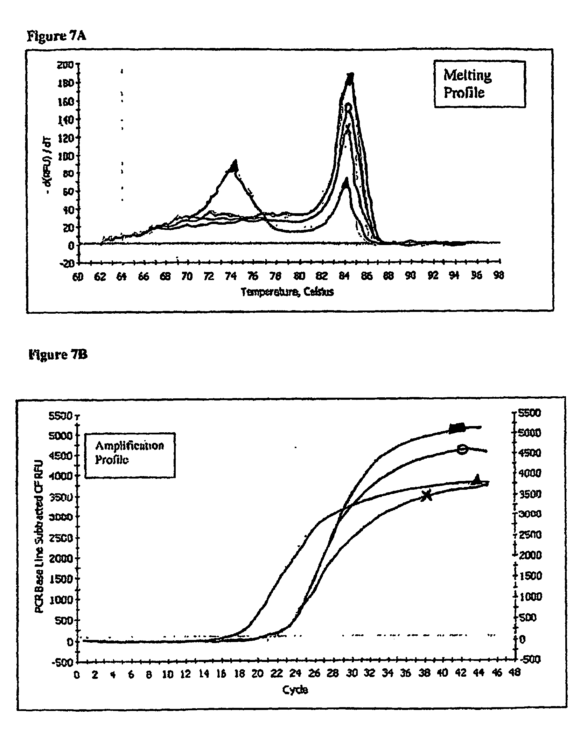

**Figure 7A** is a line graph showing the results of melting curve analysis of amplification products. The 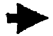 arrow shows the non-specific primer-dimer product The ⇒ arrow shows the expected amp] icon generated by amplification of the correct genomic sequence. The enzymes used for amplification and their method of preparation are shown in Table 4. **Figure 7B** is a line graph showing the amplification profile for the hot-start real time PCR analysis.

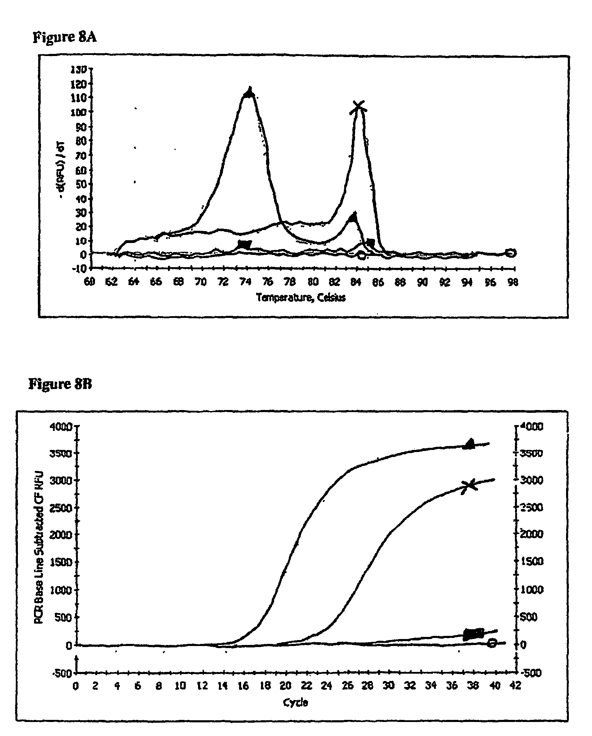

**Figure 8A** is a line graph showing the results of melting curve analysis of amplification products following the treatment of Taq DNA polymerase with DSG (in the absence of AR4 inhibitor). ■ : 5 pmole DSG/ unit of Taq; ○ : 6 pmoles DSG/unit of Taq. Control reactions of untreated Taq (◢) and Taq mixed with Taq antibodies (**X**) are also shown as controls. **Figure 8B** is a line graph showing the amplification profile for the hot-start real time PCR analysis.

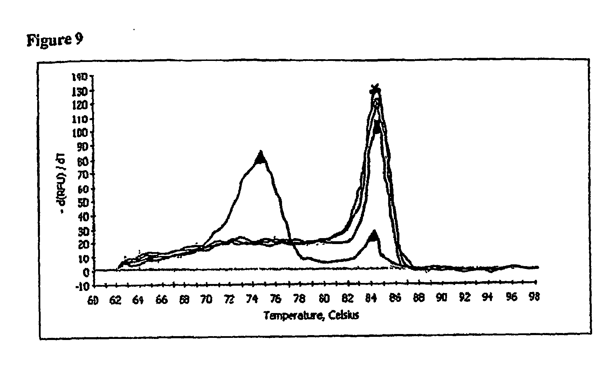

**Figure 9** is a line graph showing the results of melting curve analysis of amplification products following the conjugation of amine modified AR4 to Taq Polymerase using 4 and 6 pmoles/Unit EGS, which results in the preparation of enzymatically activatable DNA polymerase prep. ○ : 4 pmole EGS; ■ : 6 pmole EGS; **X**: AB:Taq control; ▲ : unmodified Taq.

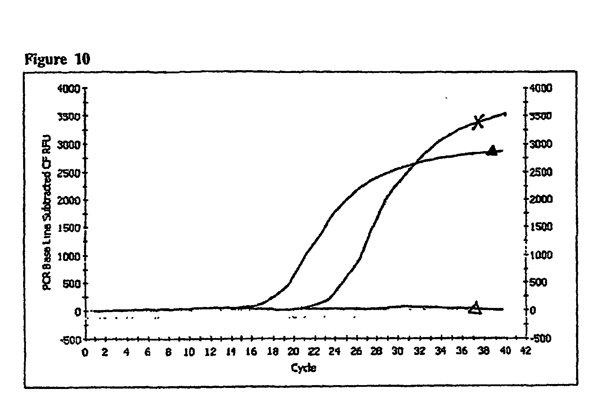

**Figure 10** is a line graph showing the results of amplification using Taq DNA polymerase combined with EGS (6 pmoles/U), which results in permanent inactivation of Taq Polymerase(Δ). Control reactions of untreated Taq (◢) and Taq mixed with Taq antibodies (x) are also shown as controls.

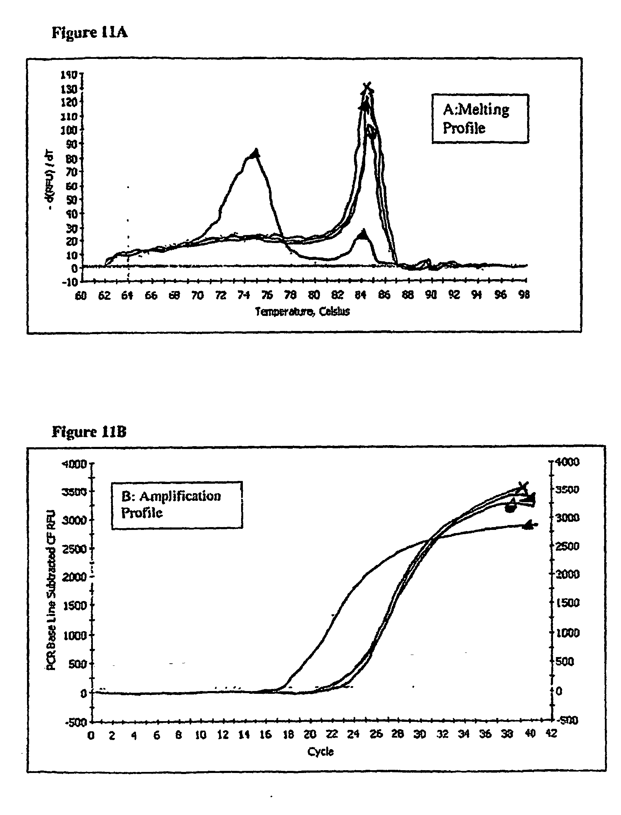

**Figure 11**A is a line graph showing the results of melting curve analysis of amplification products following the conjugation of amine modified AR4 to Taeolymerase using 1 and 2 pmoles/Unit EGS. This conjugation results in preparation of enzymatically activatable DNA polymerase. ▲ : 1pmole EGS; ◢ : 2pmole EGS; X: AB:Taq control; ▲: unmodifiedTaq. Figure 11B is a line graph showing the amplification profile for the hot-start real time PCR analysis.

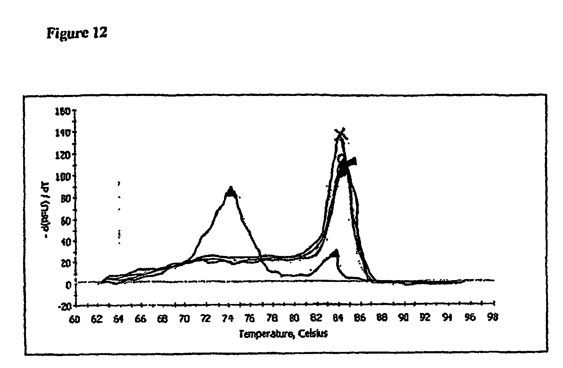

**Figure 12** is a line graph showing the results of melting curve analysis of amplification products following the conjugation of amine modified AR4 to Taq Polymerase using I and 2 pmoles/Unit EGS, which results in preparation of enzymatically activatable DNA polymerase prep. ○ : 1 pmole EGS; 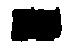 : 2 pmole EGS; X : AB:Taq control; ▲ : unmodified Taq. The conditions of conjugation were 1 pmole AR4 inhibitor, 1-2 pmoles EGS for every unit of Taq at room temperature for 30 minutes.

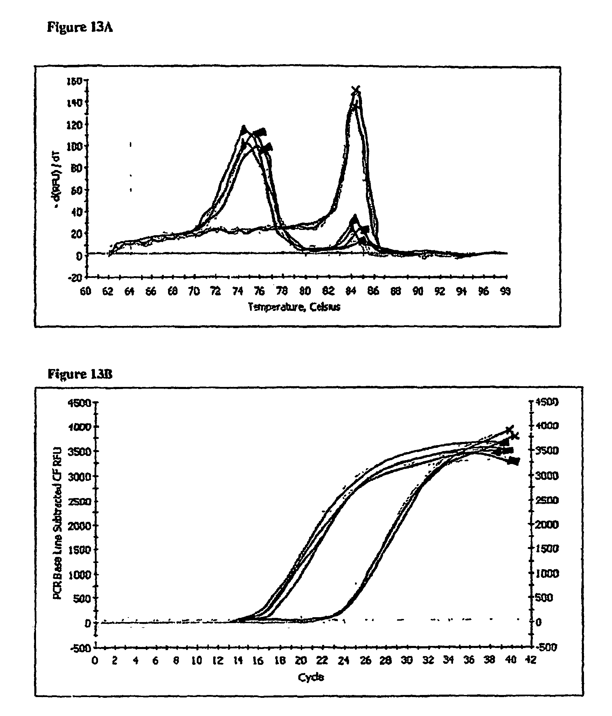

**Figure 13A** is a line graph showing the results of melting curve analysis of DNA amplification products, which demonstrates the effect of EDC on amplification. 40 pmoles of EDC were mixed with DNA polymerase in the amplification reaction and DNA polymerase activity was assessed in Q-PCR using the NDUFB primer set as described herein. Taq polymerase (▲) and AB:Taq preparation (X) without EDC were used as controls. 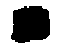 show the activity of Taq polymerase in the presence of EDC. Taq polymerase alone and mixed with EDC produced only non specific products and AB:taq mix produced the desired amplicon. **Figure 13B** is a line graph showing the amplification profile for the real time PCR analysis.

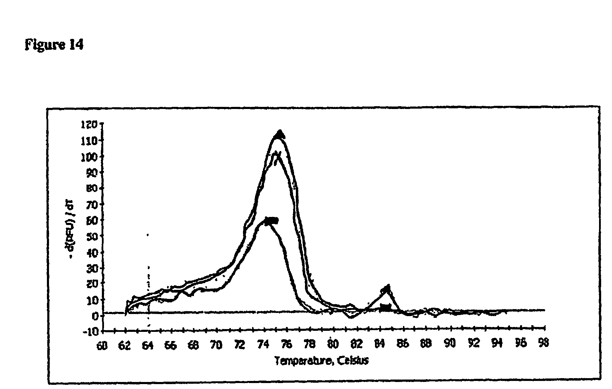

**Figure 14** is a line graph showing the results of melting curve analysis of Taq DNA polymerase amplification products, which shows the effect of incubation of Taq DNA polymerase with 40 pmoles of EDC in the absence of AR4 inhibitor for various lengths of time. ▲ : 30 min; X: 60 min; ■ : 90 min.

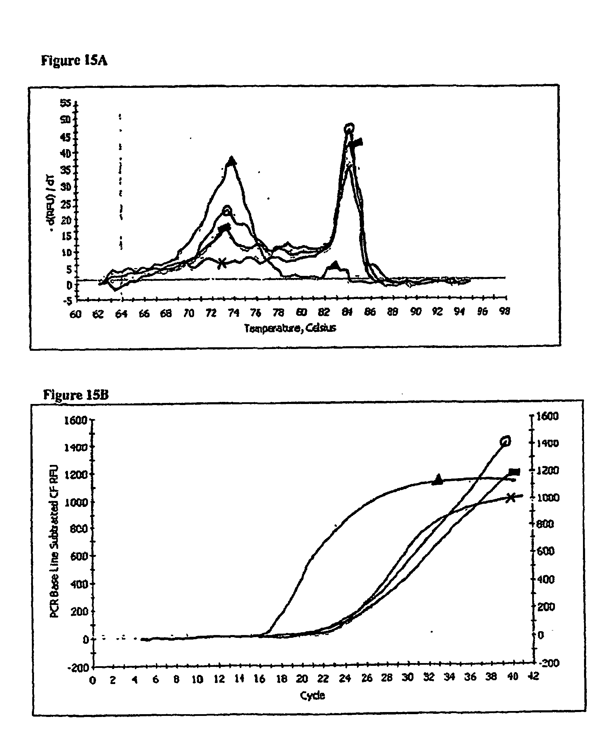

**Figure 15A** is a line graph showing the results of melting curve analysis of DNA amplification products following conjugation of various amounts of amine modified AR4 to Taq Polymerase using 10 pmoles/Unit EDC, resulting in the preparation of enzymatically activatable hotstart DNA polymerase. ○ : 0.2 pmoles AR4 and 1 hr incubation; ■ : 0.8 pmoles AR4 and 1 hr incubation; X: AB:Taq control; ▲ : unmodified Taq. **Figure 15B** is a line graph showing the amplification profile for the real time PCR analysis.

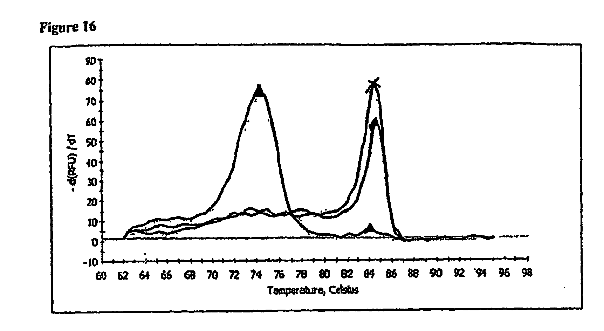

**Figure 16** is a line graph showing the results of melting curve analysis of Taq DNA polymerase amplification products following hot-start PCR using Taq DNA polymerase modified with AR4 using DSG. Tth UDG complexed with a specific monoclonal antibody was used in the reaction ( 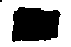 ). Control reactions with unmodified Taq (▲) and Taq antibody mediated hotstart polymerase (X) are also shown.

### DETAILED DESCRIPTION OF THE INVENTION

The instant application provides substantially improved compositions and methods for hot start nucleic acid amplification, for example hot start PCR. The DNA polymerase enzyme used for the amplification is inhibited by a binding partner that substantially inhibits polymerase activity of the enzyme and therefore prevents formation of unwanted reaction products, for example non-specific amplification products caused by polymerase activity at low temperatures, for example, ambient temperature.

In one embodiment, the binding partner, which can he covalendy or noncovalently bound to the polymerase, acts as a substrate for a second enzyme activity. That second enzyme activity modifies the binding partner in such a way that the inhibition of polymerase activity is relieved and the amplification can proceed. The activity of the second enzyme may itself be inhibited at lower temperatures such that the second enzyme activity is present only at the elevated temperatures of a thermal amplification process such as a PCR. In this way, activation of the second enzyme at elevated temperature results in removal of polymerase inhibition by the binding partner, leading to activation of the polymerase only at elevated temperature. This "hot start" procedure reduces non-specific priming and other unwanted side-reactions in the amplification process.

Also disclosed is a DNA polymerase that is covalently modified with a binding target, where the binding target is not an amino acid sequence of the polymerase. The binding target is bound by a binding moiety such that activity of the polymerase is inhibited by the presence of the binding moiety, typically through steric hindrance of the polymerase binding site or through reversible effects on the three dimensional structure of the polymerase. The binding between the binding moiety and binding target is temperature sensitive such that at elevated temperatures the binding is disrupted and the inhibition of polymerase activity is relieved. An example of a binding target is biotin, and an example of a complementary binding moiety is streptavidin or an anti-biotin antibody, although other suitable binding target/binding moiety pairs can be used as described in more detail below.

Unlike other methods, the compositions and methods of the instant invention are applicable to virtually any DNA polymerase, including archaebacterial DNA polymerases.

### Molecular Biology Terms and Definitions

In the description that follows, a number of terms used in molecular biology and nucleic acid amplification technology are extensively utilized. In order to provide a clearer and consistent understanding of the specification and claims, including the scope to be given such terms, the following definitions are provided. One of ordinary skill in the art will appreciate that the below provide definition of terms serve to guide the reader, but not limit the interpretation or understanding of the present invention. The skilled will understand the ordinary meaning of the following terms and descriptions.

"Amplification", as used herein, refers to any *in vitro* process for increasing the number of copies of a nucleotide sequence. Nucleic acid amplification results in the incorporation of nucleotides into DNA or RNA. PCR is an example of a suitable method for DNA amplification. As used herein, one amplification reaction may consist of many rounds of DNA replication. For example, one TCR reaction may consist of 10 to 50 "cycles" of denaturation and replication.

"Nucleotide" as used herein, is a term of art that refers to a base-sugar-phosphate combination. Nucleotides are the monomeric units of nucleic acid polymers (i.e., nucleic acid polymers of DNA and RNA). The term includes ribonucleoside triphosphates, such as rATP, rCTP, rGTP, or rUTP, and deoxyribonucleoside triphosphates, such as dATP, dCTP, dGTP, or dTTP. A "nucleoside" is a base-sugar combination (e.g. a nucleotide lacking phosphate).

"Exo-sample nucleotide", as used herein, refers to a nucleotide that is generally not found in a DNA sequence. For most DNA samples, deoxyuridine is an example of an exo-sample nucleotide. Although the triphosphate form of deoxyuridine, dUTP, is present in living organisms as a metabolic intermediate, it is rarely incorporated into DNA. When dUTP is incorporated into DNA, the resulting deoxyuridine is promptly removed *in vivo* by normal processes (e.g. processes involving the enzyme uracil DNA glycosylase (UDG) [Kunkel, U.S. Pat. No. 4,873,192; Duncan, B. K., The Enzymes XIV:565-586 (1981] ). As such, deoxyuridine occurs rarely or never in natural DNA. It is recognized that some organisms may naturally incorporate deoxyuridine into DNA. For nucleic acid samples of those organisms, deoxyuridine would not be considered an exo-sample nucleotide. Other examples of exo-sample nucleotides include, but are not limited to, bromodeoxyuridine, 7-methylguanine, 5,6-dihyro-5,6 dihydroxydeoxythymidine, and 3-methyldeoxadenosine. [Duncan, B.K., The Enzymes XIV:565-586 (1981)]. Still other exo-sample nucleotides will be evident to those skilled in the art. For example, RNA primers/oligonucleotides can be readily destroyed by alkali or an appropriate ribonuclease (RNase). RNase H degrades the RNA component of RNA:DNA hybrids, and numerous single-stranded RNases are known that are useful to digest single-stranded RNA after a denaturation step.

The presence of deoxyuridine, or any other exo-sample nucleotide, may be readily determined using methods well known to the art. A nucleic acid molecule containing any such exo-sample nucleotide is functionally equivalent to DNA containing only dA, dC, dG or dT (dT is referred to herein as T) in all respects, except that it is uniquely susceptible to certain treatments, such as glycosylase digestion. Numerous DNA glycosylases are known to the art. An exo-sample nucleotide which may be chemically or enzymatically incorporated into an oligonucleotide and a DNA glycosylase that acts on it may be used in conjunction with the embodiments of this invention. DNA containing bromodeoxyuridine as the exo-sample nucleotide may be degraded by exposure to light under well-known conditions.

The use of exo-sample nucleotides to remove potential contaminants from samples being subjected to PCR amplification has been previously disclosed in the art. [Longo et al., Gene 93:125-128 (1990), Hartley, U.S. Pat. No. 5,035,966 ]. Longo *et al.* and Hartley disclose the use of either dU-containing oligonucleotides or dUTP in the PCR-directed amplification of a target sequence.

Two sequences are said to be "substantially similar in sequence" if they are both able to hybridize to the same oligonucleotide.

The "terminus" of a nucleic acid molecule denotes a region at the end of the molecule. The term is not used herein as representing the final nucleotide of a linear molecule, but rather a general region which is at or near an end of a linear or circular molecule.

Two termini of two nucleic acid molecules are said to be the "same denominated termini," if the both termini are either the 3' termini of the respective molecules or both termini are the respective 5' termini of the respective molecules. As used herein, the term "same denominated termini," is not intended to refer to the nucleotide sequence of the termini being compared.

As used herein, a DNA molecule is said to be "circular" if it is capable of depiction as either a covalently closed circle, or as a hydrogen bonded circle. A circular molecule may thus be composed of one or more polynucleotides bonded to one another via covalent or hydrogen bonds. The terminal nucleotide(s) of each polynucleotide may either be single-stranded, or may be bonded to another polynucleotide via covalent or hydrogen bonds.

"Uracil DNA glycosylase" (UDG), refers to an activity that cleaves the glycosidic bond between the base uracil and the sugar deoxyribose, only when the monomeric nucleotide dUTP is incorporated into a DNA molecule, resulting in incorporation of a deoxyuridine moiety. [Duncan, B. The Enzymes 14:565 (1981), ed.: Boyer P]. An enzyme possessing this activity does not act upon free dUTP, free deoxyuridine, or RNA [Duncan, *supra*]. The action of UDG results in the production of an "abasic" site. The enzyme does not, however, cleave the phophodiester backbone of the nucleic acid molecule. The phophodiester backbone at an abasic site may be cleaved through the use of an endonuclease specific for such substrates or exposure to heat or alkaline pH. An enzyme for this purpose is the *Escherichia coli* enzyme, Endonuclease IV. Endonuclease IV can be used in conjunction with UDG to remove dU residues from a nucleic acid molecule.

"Incorporating" as used herein, means becoming part of a nucleic acid polymer.

"Terminating" as used herein, means causing a treatment to stop. Termination includes means for both permanent and conditional stoppages. For example, if the treatment is enzymatic, a permanent stoppage would be heat denaturation. An example of a conditional stoppage would be the use of a temperature outside the enzyme's active range. Both types of termination are intended to fall within the scope of the embodiments of the present invention.

"Oligonucleotide" as used herein refers collectively and interchangeably to two terms of art, "oligonucleotide" and "polynucleotide". Note that although oligonucleotide and polynucleotide are distinct terms there is no exact dividing line between them and they are used interchangeably herein. An oligonucleotide is said to be either an adapter, adapter/linker or installation oligonucleotide (i.e., the terms are synonymous) if it is capable of installing a desired sequence onto a predetermined oligonucleotide. An oligonucleotide may serve as a primer unless it is "blocked.". An oligonucleotide is said to be "blocked," if its 3' terminus is incapable of serving as a primer.

"Oligonucleotide-dependent amplification" as used herein refers to amplification using an oligonucleotide or polynucleotide to amplify a nucleic acid sequence. An oligonucleotide-dependent amplification is any amplification that requires the presence of one or more oligonucleotides or polynucleotides that are two or more mononucleotide subunits in length and that end up as part of the newly formed, amplified nucleic acid molecule.

"Primer" as used herein refers to a single-stranded oligonucleotide or a single-stranded polynucleotide that is extended by covalent addition of nucleotide monomers during amplification. Nucleic acid amplification often is based on nucleic acid synthesis by a nucleic acid polymerase. Many such polymerases require the presence of a primer that can be extended to initiate such nucleic acid synthesis. A primer is typically 11 bases or longer, advantageously 17 bases or longer. A primer will contain a minimum of 3 bases.

A "probe" is an oligonucleotide which is substantially complementary to a nucleic acid sequence of the target nucleic acid and which is generally not allowed to form primer extension products. Probes can be labeled, usually at the 3' end, with any suitable detectable material, as described below. They can also be attached to a water-insoluble substrate of some type for capture of the targeted nucleic acid using known technology.

"Reaction volume" denotes a liquid suitable for conducting a desired reaction, such as an amplification, hybridization, cDNA synthesis, etc. When an enzymatic reaction, such as a ligation or a polymerization reaction, is being conducted, it is preferable to provide the components required for such reaction in "excess" in the reaction vessel. "Excess" in reference to components of the amplification reaction refers to an amount of each component such that the ability to achieve the desired amplification is not limited by the concentration of that component.

It is to be understood that this invention is not limited to the particular methodologies, protocols, constructs, formulae and reagents described and as such may vary. It is also to be understood that the terminology used herein is for the purpose of describing particular embodiments only, and is not intended to limit the scope of the present invention.

It must be noted that as used herein and in the appended claims, the singular forms "a," "and," and "the" include plural reference unless the context clearly dictates otherwise. Thus, for example, reference to "a peptide" is a reference to one or more peptides and includes equivalents thereof known to those skilled in the art, and so forth.

Unless defined otherwise, all technical and scientific terms used herein have the same meaning as commonly understood to one of ordinary skill in the art to which this invention belongs. Although any methods, devices, and materials similar or equivalent to those described herein can be used in the practice or testing of the invention, the preferred methods, devices and materials are now described.

As used herein in referring to primers, probes or oligomer fragments to be detected, the term "oligonucleotide" refers to a molecule made of two or more deoxyribonucleotides or ribonucleotides. Its exact size is not critical, but depends upon many factors including the ultimate use or function of the oligonucleotide. The oligonucleotide may be derived by any method known in the art.

Amplification refers to an increase in the amount of the desired nucleic acid molecule present in a sample. An "amplification reagent" refers to any of the reagents considered essential to nucleic acid amplification, namely one or more primers for the target nucleic acid, a thermostable DNA polymerase, a DNA polymerase cofactor, and one or more deoxyribonucleoside-5'-triphosphates.

A primer is typically a single-stranded oligonucleotide or a single-stranded polynucleotide that is extended by covalent addition of nucleotide monomers during amplification. The primer can also contain a double-stranded region if desired. Nucleic acid amplification often is based on nucleic acid synthesis by a nucleic acid polymerase. Many such polymerases require the presence of a primer that can be extended to initiate such nucleic acid synthesis. The primer must be long enough to prime the synthesis of extension products in the presence of the DNA polymerase. The exact size of each primer will vary depending upon the use contemplated, the complexity of the targeted sequence, reaction temperature and the source of the primer. The primers used in the present invention may be substantially complementary to the target strands of each sequence to be amplified. This means that they must be sufficiently complementary to hybridize with their respective strands to form the desired hybridized products and then be extendable by a DNA polymerase.

Primers useful herein can be obtained from a number of sources or prepared using known techniques and equipment, including for example, an ABI DNA Synthesizer (available from Applied Biosystems) or a Biosearch 8600 Series or 8800 Series Synthesizer (available from Milligen-Biosearch, Inc.) and known methods for their use. For example, as described in U.S. Pat. No. 4,965,188. Naturally occurring primers isolated from biological sources are also useful, such as restriction endonuclease digests. Additionally, the term "primer" also refers to a mixture of different primers.

As used herein, two sequences are said to be able to hybridize or anneal to one another if they are capable of forming an anti-parallel double-stranded nucleic acid structure. Conditions of nucleic acid hybridization suitable for forming such double-stranded structures are known. Thus, the sequences need not exhibit precise complementarity, but need only be sufficiently complementary in sequence to be able to form a stable double-stranded structure. Departures from complete complementarity are permissible, so long as such departures are not sufficient to completely preclude hybridization to form a double-stranded structure.

Hybridization of a primer to a complementary strand of nucleic acid is a prerequisite for its template-dependent polymerization with polymerases. Factors that affect the base pairing of primers to their complementary nucleic acids subsequently affect priming efficiency (i.e., the relative rate of the initiation of priming by the primer). The nucleotide composition of a primer can affect the temperature at which annealing is optimal and therefore can affect its priming efficiency. [Maniatis et al., "Nucleic Acid Hybridization, A Practical Approach," IRL Press, Washington, DC (1985)].

Additional methods generally useful in adjusting the hybridization efficiency of the primers used to amplify a nucleic acid molecule have been described elsewhere. See Schuster et al. - U.S. Pat. No. 5,869,251.

### Enzyme inhibition using an inhibitor that is an enzyme substrate

The present invention provides a composition as defined in the claims containing (1) a thermostable DNA polymerase and (2) an inhibitor for the DNA polymerase, where the inhibitor binds to and inhibits the enzymatic activity of the DNA polymerase. The inhibitor is irreversibly inactivated by activity of a second enzyme such that the DNA polymerase regains its enzymatic activity. The inhibitor can be bound to the polymerase covalently or non-covalently.

The skilled artisan will recognize that any inhibitor that can be suitably modified by a second enzyme activity is suitable for use in the present invention. Suitable inhibitors can be single or double stranded nucleic acids, polypeptides, polypeptides modified by post-translational modifications such as phosphorylation and glycosylation, lipids, and the like. Specific examples are described in more detail below.

In a particular embodiment, the inhibitor can be a nucleic acid that contains one or more exo-sample nucleotides, and the second enzyme is an enzyme that recognizes and modifies such nucleic acids. A specific example is a nucleic acid inhibitor that contains one or more uracil nucleotides that are recognized by a uracil DNA glycosylase.

Also diclosed is a kit for polymerase chain reaction comprising, in separate packaging or the like, a composition comprising (1) a thermostable DNA polymerase, (2) an inhibitor for the DNA polymerase, wherein the inhibitor is capable of inhibiting the enzymatic activity of the DNA polymerase and the inhibitor is irreversibly inactivated by a thermostable uracil DNA glycoslyase ("UDG") such that the DNA polymerase regains its enzymatic activity, and (3) at least one additional PCR reagent.

Also diclosed is a kit comprising, in separate packaging or the like, (1) a thermostable DNA polymerase, (2) an inhibitor for the thermostable DNA polymerase, (3) a thermostable uracil DNA glycosylase ("UDG"), and (4) a temperature sensitive inhibitor of the thermostable UDG. In a specific embodiment of the invention, the thermostable DNA polymerase inhibitor is a DNA fragment greater than 3 nucleotides in length and containing at least one deoxyuridine residue ("dU"). UDG is an enzyme that is capable of degrading the inhibitor nucleic acid containing dU. The temperature sensitive inhibitor for UDG is an antibody, or portion of an antibody, that is capable of binding to UDG at ambient temperatures and is irreversibly inactivated at higher temperatures.

Also diclosed is a method for the amplification of a target nucleic acid comprising the steps of contacting a specimen, suspected of containing a target nucleic acid, with (1) a primer complementary to the target nucleic acid, (2) a thermostable DNA polymerase, (3) an inhibitor for the thermostable DNA polymerase, (4) a thermostable UDG, and (5) a temperature sensitive inhibitor for UDG, and bringing the resulting mixture to a temperature, wherein the UDG inhibitor is inactivated and allows UDG to degrade the thermostable DNA polymerase inhibitor, thereby allowing the formation of primer extension products. In a specific embodiment, the inhibitor for the thermostable DNA polymerase is capable of binding with the polymerase at about temperature T1, wherein T1 is a temperature at which the enzymatic activity of the DNA polymerase is inhibited. In a more specific embodiment, the inhibitor for the thermostable DNA polymerase is capable of binding with the polymerase at about temperature T1, wherein T1 is less than about 50°C. In one embodiment of the invention, UDG is capable of degrading the dU-containing DNA fragment at high temperatures and thereby activates the thermostable DNA polymerase for PCR amplification.

In one embodiment, an antibody is provided that is specific to a thermostable UDG. In another embodiment of the invention, the UDG-specific antibodies are capable of binding to UDG at about temperature T1 and are irreversibly inactivated at about temperature T2 so that UDG regains its enzymatic activity. Methods for generating antibodies against a protein of choice are well known in the art.

It also is well known in the literature that there are natural proteins and peptides that are inhibitors of UDG and these commonly known inhibitors may be used in accordance with the methods of this invention. For example, one such inhibitor is uracil glycosylase inhibitor ("UGI") that is capable of binding some uracil DNA glycosylases.

The UDG enzyme of the mesophilic organism *Escherichia coli (E. coli*) has been studied most extensively, and the gene encoding the protein (ung) has been cloned. See Varshney et al., 1988. J. Biol. Chem., 263:7776-7784]. The UDG genes of herpes simplex virus type-1, *Haemophilus influenzae* [1995. Science 269(5223):496-512], *Streptococcus pneumonia* [1990. Nucl. Acids Res. 18(22):6693) and *Bacillus subtilis* [1993. Mol. Microbiol. 10(2):371-384] have also been isolated and sequenced. Thermophilic UDG proteins have been isolated from the thermophilic bacteria *Bacillus stearothermophilus* and *Thermothrix thiopara,* which have optimum temperatures for growth of 55°C and 75°C, respectively. [O. K. Kaboev, et al. 1981. FEBS Lett. 132:337-340; O. K. Kaboev, et al. 1985. J. Bacteriol. 164:421-424]. United States Patent No. 5,888,795 describes cloning and expression of a thermostable UDG from *Bacillus pallidus.* The UDG genes are fairly homologous, however, in spite of sequence divergence demonstrated by hybridization studies and sequence analysis, the tertiary structure of the UDG enzymes has been found to be highly conserved. [Varshney *et al.,* supra]. Numerous uracil glycosylase enzymes have been isolated from a variety of organisms including thermophilic microorganisms. For example, Sandigursky and Franklin have cloned and expressed a thermostable UDG from *Thermatoga maritima.* [Current Biology, 1999, Vol. 9: 531-434]. Additionally, a number thermostable UDG enzymes have been cloned and expressed from *Thermus thermophilus* [Starkuviene, 2001, Doctoral Dissertation, University of Gottingen].

The *Bacillus subtilis* bacteriophage PBS1 is unique in that it incorporates deoxyuracil instead of thymine in its DNA. This phage must therefore protect itself from a host cell UDG upon infection. To do so, PBS1 produces a uracil glycosylase inhibitor protein ("UGI"), which complexes with UDG thereby rendering the UDG inactive. The PBS1 gene encoding UGI, and genes from closely related *Bacillus subtilis* PBS phages such as PBS2, have been cloned and expressed to produce recombinant UGI. The UGI protein has been shown to be an effective means for controlling residual UDG activity still present after heat inactivation in PCR [Rashtchian et al., Biotechniques, Vol. 13, No. 2, page 180]. It has further been shown that UGI alone is effective to inactivate UDG in isothermal amplification reactions such as strand displacement amplification ("SDA"), which do not have high temperature cycling and which may be incompatible with high temperature steps for inactivation of UDG.

The present invention overcomes the problem of amplification of non-target nucleic acids by inactivating the DNA polymerase at low temperatures and controlling the polymerase activity until the reaction conditions are desirable (i.e. higher temperatures). The formation of primer dimers is also greatly reduced by the methods of the present invention.

The advantages of the present invention are achieved by the method of mixing a thermostable DNA polymerase with an inhibitor for the DNA polymerase. This inhibitor binds the thermostable DNA polymerase and results in inhibition of polymerase activity. This inhibition is reversible by enzymatic digestion of the nucleic acid inhibitor at about temperature T2 by UDG. In other words, the inhibitor binds the DNA polymerase until the reaction reaches about temperature T2, wherein the antibody inhibiting the UDG is inactivated allowing the UDG to degrade the dU-containing DNA fragment and thereby activating the DNA polymerase for amplification. As described above a number of UDG's have been described and can be used according to the methods of invention.

Employing the methods of the instant application, it is possible to control amplification methods such as PCR by keeping the temperature of the reaction comprising DNA polymerase, dU-containing DNA fragment, UDG, and an inhibitor for UDG at temperature T1, and then let the reaction proceed by raising the temperature of the reaction mix to at least temperature T2. These procedures provide a very effective and convenient hot start method for amplification.

A thermostable DNA polymerase is heat-stable and preferentially active at higher temperatures, especially the high temperatures used for denaturation of DNA strands. More particularly, the thermostable DNA polymerases are not substantially inactivated at the high temperatures used in polymerase chain reactions as described herein. Such temperatures will vary depending upon a number of reaction conditions, including pH, the nucleotide composition of the target nucleic acid and primers, the length of primer, salt concentration and other conditions known in the art. DNA polymerase catalyzes (e.g. facilitates) the combination of the nucleotides in the proper manner to form the primer extension products that are complementary to each nucleic acid strand.

Examples of enzymes that have been reported in the literature as being resistant to heat include heat-stable polymerases, such as those extracted from the thermostable bacteria *Thermus flavus, Thermus ruber, Thermus thermophilus, Bacillus stearothermophilus, Thermus aquaticus, Thermus lacteus, Thermus rubens,* and *Methanothermus fervidus.* The thermostable enzyme may be produced by recombinant DNA techniques by the method described U.S. Pat. No. 4,889,818. The thermostable enzyme also may be stored stably in a buffer. [U.S. Pat. No. 4,889,818].

In a specific embodiment of the present invention, the activity of DNA polymerase is inhibited by a form of deoxyuracil ("dU"). The ability of dU-containing nucleic acids to bind to DNA polymerase allows one to control amplification using such polymerases to overcome problems discussed above with such amplification procedures as PCR. By allowing dU-containing DNA to bind DNA polymerase, and later degrading the dU, control over amplification resulting in an increased yield of specific PCR product is achieved. The present invention may use dU as an inhibitor as part of a DNA fragment that is usually greater than three nucleotides in length. The dU-containing DNA fragment can be single-stranded, double-stranded, or partially single- and partly double-stranded. Nucleic acid fragments containing dU can be made synthetically or enzymatically using commercially available reagents and instruments. The dU-containing DNA fragments can also be made in vivo as described by Kunkle (US patent 4873192).

The inhibitor molecule can be a modified nucleic acid that can inhibit activity of DNA polymerase. DNA containing dU is only an example of modification of a nucleic acid that is useful for the present invention. Depending on the modified nucleic acid used for inhibition, an appropriate enzyme will be needed for removal of the inhibitor at the desired temperature to effect hot start polymerization reaction. Other exo-sample nucleotides are known in the literature and appropriate enzymes have been isolated to specifically degrade such exo-sample nucleotide containing nucleic acids without affecting sample DNA. Other DNA glycosylases are known in the art and may be used in accordance with the methods of the present invention. [Duncan, B. The Enzymes, 14:565 (1981), ed.: Boyer P].

Examples of other modified nucleotides are hypoxanthine, 5-methyl cytosine, 3-methyl adenine and 7-methylguanine. A wide variety of enzymes have been described that have evolved to recognize and degrade modified bases or mismatched bases in DNA. (Sartori et al., Embo J. Vol.12: 3182-3191, 2002; Aravind et al. Genome Biology 2000, 1(4):research 0007.1-0007.8 ;Wyeth et al, BioEssays 21:668-676, 1999) These modified nucleic acids and appropriate enzymes that bind to them or modify them can be used according to the methods of the present invention. In other words, any modified nucleic acid can be used as a reversible inhibitor for polymerases and an appropriate specific enzyme can be used to selectively remove or modify the inhibitor molecules and therefore activate the polymerase under a desirable condition. A nucleic acid used as an inhibitor may have a blocked 3' OH group so that it cannot serve as a primer.

In other specific embodiments of the present invention, the inhibitor nucleic acid need not be modified with other nucleotides. The inhibitor can be differentiated from sample DNA by nature of its nucleotide sequence and use of specific enzymes that recognize those sequences. For example restriction enzyme sites can be incorporated into the inhibitor DNA and restriction enzymes can be used to remove the inhibitor for activation of the polymerase. Other nucleases that specifically recognize a particular feature in nucleic acids and modify or digest such nucleic acids can be used in accordance with the methods of present invention. Lambda exonuclease for example digests only DNA molecules that have a 5' end phosphate. Enzymes such as Mut L or Mut S and related proteins recognize mismatches in double stranded molecules and cleave them.

In other specific embodiments of the present invention, RNA molecules can be used as inhibitors. The RNA inhibitor can be composed of ribonucleotides or can be a chimera of RNA and DNA. If an RNA-containing inhibitor is used, an enzyme with ribonuclease activity can be used for removal of the inhibitor at the desired temperature or condition to activate the DNA polymerase. Inhibitors of ribonucleases are known in the literature. RNase inhibitor proteins specifically bind to RNases and inhibit their activity. For example, ribonuclease A is known to be thermostable and can bind to RNase inhibitor protein at ambient temperatures. However, because RNase inhibitor protein is thermolabile, it can be used as a thermolabile inhibitor of Ribonuclease. At ambient temperatures ribonuclease will be inactive, and the RNA containing nucleic acid inhibitor inhibits the polymerase activity. Upon increasing the temperature during the first cycle of PCR, RNase inhibitor will be heat inactivated, releasing active ribonuclease. In turn, the ribonuclease will degrade the RNA inhibitor of the polymerase and result in activation of DNA polymerase activity. This cascade of events provides a new method hot start PCR and results in improved specificity of PCR. Ribonucleases and RNase inhibitor proteins are available commercially and many such proteins have been described in the literature from a variety of sources. [Blackburn et al., J. Biol. Chem., Vol 252,5904-5910, 1977; Lee et al., Biochemistry 28: 225-230; Klink et al., Protein Expression and Purification, 22: 174-179, 2001]. In one specific example, the inhibitor can be a so-called "gap-mer" RNA-DNA hybrid that contains a region of RNA-DNA duplex that is a substrate for RNAseH, and RNAseH is the enzyme that is used for removing the inhibitor.

A DNA polymerase cofactor refers to a nonprotein compound on which the enzyme depends for activity. Thus, the enzyme may be catalytically inactive, or the activity greatly reduced, without the presence of the cofactor. A number of such materials are known cofactors including manganese and magnesium compounds. Such compounds contain the manganese or magnesium in such a form that divalent cations are released into an aqueous solution. Useful cofactors include, but are not limited to, manganese and magnesium salts, such as chlorides, sulfates, acetates and fatty acid salts, for example, butyric, caproic, caprylic, capric and lauric acid salts. The smaller salts, such as chlorides, sulfates and acetates are preferred.

Also needed for amplification is a source of deoxyribonucleoside-5'-triphosphates, such as dATP, dCTP, dGTP, dTTP or dUTP. Analogues such as dITP and 7-deaza-dGTP may also be useful. dATP, dCTP, dGTP and dTTP collectively are often referred to as dNTP's.

Uracil-DNA-glycosylases are wide-spread, highly conserved and extremely specific DNA repair enzymes. Their biological function is to specifically remove the base uracil from DNA. This enzyme cleaves the glycosidic bond between the base uracil and the sugar deoxyribose, only when the monomeric nucleotide dUTP is incorporated into a DNA molecule, resulting in incorporation of a deoxyuridine moiety. The enzyme does not act upon free dUTP, free deoxyuridine, or RNA.

In an embodiment of the invention, the activity of the UDG may be inhibited using an antibody, or a portion of an antibody, that binds to the UDG molecule in such as way as to interfere with its function. The antibodies used in this invention may be polyclonal, monoclonal, or chimeric antibodies, single-chain antibodies, or may be any portion of an antibody that binds to UDG, such as an antibody fragments including, but not limited to F(ab')₂, F(ab)₂, Fab', Fab, and the like. The portion of the antibody may be made by fragmenting an antibody, or the portion may be produced recombinantly or synthetically. The generation of all of the above-mentioned antibodies are well-known in the art. Antibodies that bind UDG may also be obtained commercially, from, e.g., Pharmingen, a unit of BD Biosciences (San Diego, CA).

Also disclosed is a method for the amplification of a target nucleic acid comprising the steps of contacting a specimen suspected of containing a target nucleic acid with (1) a primer complementary to the target nucleic acid, (2) a thermostable DNA polymerase, (3) an inhibitor for the thermostable DNA polymerase, (4) a thermostable UDG, and (5) a temperature sensitive inhibitor for UDG, and bringing the resulting mixture to about T2, wherein the UDG inhibitor is inactivated and allows UDG to degrade the thermostable DNA polymerase inhibitor which allows the formation of primer extension products.

In a preferred embodiment of the present invention, the inhibitor of the thermostable DNA polymerase is a DNA fragment greater than three nucleotides containing at least one deoxyuracil residue and is capable of binding with the polymerase at about temperature T1, where T1 is a temperature at which the enzymatic activity of the DNA polymerase is inhibited.

UDG is capable of degrading the dU-containing DNA fragment at about temperature T2 (due to inactivation of the antibody inhibitor for UDG) and thus activates the thermostable DNA polymerase for amplification.

It should be noted that the inhibitor nucleic acid molecule can be simply mixed with the DNA polymerase and bind to polymerase due to affinity of DNA polymerase to nucleic acids. The inhibitor nucleic acid can be single or double stranded. It can also be partially double stranded with a single stranded region(s). The nucleic acid inhibitors of the present invention may DNA, RNA or RNA:DNA hybrids. When a RNA: DNA hybrid is used, RNAse H or proteins with RNAse H activity may be used for restoring the polymerase activity.

It is also possible to use nucleic acids that consist of both deoxyribonucleosides and ribonucucleoside bases. Such inhibitors may be cleaved by DNAses or RNases or both. If these inhibitors have double stranded regions that consist of RNA: DNA hybrids then they also are cleavable by RNase H. Rnase H activity has been detected in variety of organisms and viruses. Rnase H enzymes are commercially available from a variety of commercial sources. Thermostable RNAse H enzymes have also been described and are available commercially (Epicenter, Inc. , Madison, WI).

The nucleic acid inhibitor can be covalently or non covalently attached to the polymerase molecule. There are numerous methods for attachment of nucleic acids to proteins and various reagents are available commercially for modification of nucleic acids with a variety of reactive groups. US patents 4,873,187 and 6,326,136 describe methods of linking DNA probes to proteins and their use in hybridization. Different amino acids in proteins have reactive groups such as amines, thiols or carboxyls which can be utilized for attachment of various compounds including nucleic acids. All such groups can be utilized according to the methods of invention. The amine group of lysine residues are often good reactive groups for such modifications or attachments.

The present invention provides methods and compositions or reversibly inhibiting one or more activities of a DNA polymerase and specific modification or removal of the inhibiting moiety or molecule by enzymatic methods. The inhibitory binding molecule is contacted with the polymerase and can substantially reduce or inhibit the polymerase activity. Use of an appropriate enzyme that is active at higher temperatures modifies the inhibiting molecule so that it is no longer inhibitory to polymerase, restoring polymerase activity.

The methods of the invention can be applied using a variety of binding moieties and partners. The binding partner can be a specific protein or peptide that can be contacted with polymerase and can be enzymatically modified or cleaved by a protease. There are proteases in the literature that specifically recognize, bind and cleave particular peptide sequences, advantageously peptide sequences that are relatively rare and do not occur in DNA polymerase enzymes. One such protease is TEV protease which is available commercially(Invitrogen Corp.).

Attachment of a variety of moieties and molecules to protein has been described in the literature. It has also been shown that the lysine groups of Taq DNA polymerase can be chemically modified to produce an inactive Taq DNA polymerase (Birch etal. US patents 5,773,258 and 5,677,152). These patents describe a reversible modification that restores the chemical nature of lysine residues to original state after prolonged incubation at high temperatures. The linkage methods to lysines and other reactive groups can be used with embodiments and according to the methods of the present invention. Enzymatically modifiable binding partners can be covalently bound to the polymerase and, depending on the nature and relative concentration of the binding partner used used the activity of polymerase can be modulated. In cases where linkage results in inactivation of polymerase activity the inhibitor can be treated with a binding partner that modifies the inhibitor and results in restoration of polymerase activity. According to the present invention the inhibitor moiety or molecule can be selected from a plurality of compounds and used in combination with an appropriate binding partner.

By way of example the modification agent can be a lipid that is covalently attached to the polymerase and that is modified by the action of a lipase. Methods for linking lipids to polypeptides are known in the art, and suitable lipase enzymes also are known. A variety of carbohydrates can be used for modification and used in combination with enzymes that bind and modify carbohydrates.

In other embodiments of the present invention nucleic acids that are self cleaving, such as ribozymes, can be used for modification of the polymerase molecule. When such modification agents are used they may self cleave under appropriate conditions and or can be used with other enzymes that are capable of cleaving or modifying them. It should be noted that the above exemplified modifying agent or a binding partner can be combined and more than one binding partner can be used according the methods of the invention. The modification of the polymerase molecule can be accomplished by engineering of new sequences or domains to the polymerase gene and expression of new and modified polymerases that can be used according to the present invention. For example new domains and peptides can be added to the gene with a site specific protease site such as TEV protease site. Use of such enzyme with TEV protease will result in activation of the polymerase.

In other embodiments of the present invention, the polymerase molecules can be modified using the methods described above. Depending on the degree of modification and /or the nature of the modifying agents, polymerase activity of the enzyme may not be affected. In these cases, the invention provides method for reversible inhibition of polymerase activity. For example the surface of the polymerase can be modified with biotin or other antigenic determinants and be reacted with one or more binding reagents, such as antibodies, that are specific to biotin or the antigenic determinant used. Binding of the antibody to polymerase through the specific antigen can result in reversible inactivation of the polymerase. At high temperatures the antibodies will denature and polymerase activity will be restored.

Using this method a variety of binding partners can be used and the method is not confined to antigens and antibodies. A variety of peptide sequences are known in the literature that are specifically recognized by other proteins (binding partners). Essentially all enzymes and their substrates are binding partners and can be used according to the methods of the present invention. Specific domains or peptides can also be engineered into gene sequences and such recombinant proteins with a recognizable domain or peptide can bind to appropriate proteins or binding partners. There are many such domains or peptides reported in the literature. Maltose binding protein (New England BioLabs, Beverly, MA) has been fused to many proteins and His tag has been used extensively (Qiagen). The binding partner for such modifications can be used according to the methods of the invention.

In addition to the natural binding partners that can be used, other binding targets and binding partners can be developed by molecular evolution. DNA and RNA molecules have been identified and evolved to bind and cleave DNA or RNA substrates( DNAzymes or RNAzymes, Proc. Natl. Acad. Sci. USA Vol. 94, pp. 4262-4266, April 1997; GF Joyce, Annu Rev Biochem. 2004;73:791-836.). Such methods can be used to evolve new binding partners that can be thermolabile and be used according to the methods and compositions of the present invention.

In one embodiment where UDG is used to degrade dU containing polymerase inhibitor, UDG is kept inactive with an antibody at low temperatures. The antibody(ies), or a portion thereof, can be either monoclonal or polyclonal. The antibodies are temperature sensitive and are capable of binding to the thermostable UDG at about temperature T1 and are irreversibly inactivated at about temperature T2. The inactivation of the antibodies by raising the temperature of the mixture allows UDG to regain its enzymatic activity.

Depending on the UDG used to activate the hotstart enzyme preparation and its activity profile it is possible that an inhibitor would not be needed. It is well known in the art that different enzymes have different temperature profile of activity. Accordingly, an enzyme having no or very little activity at ambient temperatures but that is highly active at higher temperature can be used. It is also well documented that through site directed mutagenesis or molecular evolution it is possible to obtain an enzyme with defined characteristics useful for the methods of the present invention.

Alternatively the inhibitor nucleic acid may be protected from UDG action by means of masking the inhibitor nucleic acid at lower temperatures. For example, the dU inhibitor DNA can be masked with DNA binding proteins which would protect the DNA from digestion with UDG at low temperatures. Examples of such DNA binding proteins could be , but not limited to, single strand binding proteins (SSB), RNA polymerases that recognize promoter sequences, antibodies that bind to single stranded DNA or double stranded DNA. The inhibitor nucleic acid can also be modified with antigenic determinants and specific antibodies against these moieties can be used to sterically protect the inhibitor from cleavage at temperatures that are not desirable. Since all these masking proteins can be obtained from mammalian sources or mesophilic or psychrophilic organisms, they would not be thermostable and at higher temperature would be dissociated from the inhibitor DNA and make it available for cleavage/digestion by thermostable UDG or other nucleases that are used for activation of polymerase. The masking molecules also need not be proteins and can be simpler moieties. For example it has been shown that spermidine and spermine can bind single and double stranded DNA under physiological conditions. Also cationic lipids, used to deliver nucleic acids into cells by transfection, bind DNA and RNA. Such chemical molecules can also be used for masking of the inhibitor DNA and therefore protecting it from digestion or cleavage by the enzyme used for hotstart at low temperatures.

In a specific embodiment, the teachings of the present invention are combined with other processes in the arts of molecular biology to achieve a specific end. For example, the target sequence may be purified from the other sequences in the sample, which can be accomplished by annealing the nucleic acid sample to an oligonucleotide complementary to the target that is immobilized on a solid support. One example of a convenient solid support is a micro-bead. In a preferred embodiment the micro-bead is a magnetic micro-bead. Examples of other supports are known to those skilled in the art. After being bound, the non-target sequences may be washed away, resulting in a complete or a partial purification of the target sequence.

Amplification products may be further purified by gel electrophoresis, column chromatography, affinity chromatography, or hybridization, etc. The fractions containing the purified products may be subjected to further amplification in accordance with the methods of the invention.

Additionally, amplification products may be detected by any number of techniques known in the art. For example, amplification products can be captured by an oligonucleotide complementary to a sequence determined by the target sequence, the oligonucleotide being bound to a solid support such as a magnetic micro-bead. Preferably, this oligonucleotide's sequence does not overlap with that of any oligonucleotide used to purify the target before the amplification. RNA:DNA hybrids formed may then be detected by antibodies that bind RNA:DNA heteroduplexes. The bound antibody is then detected by a number of methods well known to the art. Alternatively, amplified nucleic acid can be detected by gel electrophoresis, hybridization, or a combination of the two, as is well understood in the art. Those in the art will recognize that the present invention can be adapted to incorporate many detection schemes.

The present invention includes articles of manufacture, such as kits. Such kits will, typically, be specially adapted to contain in close compartmentalization, each container holding a component useful in carrying out amplification according to the methods and compositions taught herein.

### Nucleic acids that may be amplified using the methods of the invention

The present invention provides for improved amplification of one or more specific nucleic acid sequences present in one or more target nucleic acids in a test specimen. Such specimens can include biological samples including cellular or viral material, hair, body fluids, tissue samples, foodstuffs, or other materials containing detectable genetic DNA or RNA. Although the primary purpose of detection is diagnostic in nature, the invention can also be used to improve the efficiency of cloning DNA or messenger RNA, or for obtaining large amounts of the desired sequence from a mixture of nucleic acids resulting from chemical synthesis.

The present invention provides for the amplification of a desired nucleic acid molecule, such as DNA or RNA, in a sample, may be used to amplify any desired nucleic acid molecule. The nucleic acid molecule may be in either a double-stranded or single-stranded form, and double-stranded nucleic acids may be denatured by any number of methods known in the art. [see, e.g., Maniatis, Molecular Cloning: A Laboratory Manual, Cold Spring Harbor Laboratories, Cold Spring Harbor, NY (1982)].

The nucleic acid molecules that may be amplified in accordance with the present invention may be homologous to other nucleic acid molecules present in the sample. For example, it may be a fragment of a human chromosome isolated from a human cell, tissue, biopsy, *etc.* Alternatively, the molecule may be heterologous to other nucleic acid molecules present in the sample. For example, it may be a viral, bacterial, or fungal nucleic acid molecule isolated from a sample of human blood, stools, fluids, etc. The methods of the invention are capable of simultaneously amplifying both heterologous and homologous molecules. For example, amplification of a human tissue sample infected with a virus may result in amplification of both viral and human sequences.

Nucleic acids to be detected can be obtained from various sources including plasmids and naturally occurring DNA or RNA from any source , including, but not limited to bacteria, yeast, viruses, plants and higher animals, humans. It may be extracted from various tissues including blood, peripheral blood mononuclear cells ("PBMC"), tissue material or other sources known in the art using known procedures. The present invention is particularly useful for the amplification and detection of nucleic acid sequences found in genomic DNA, bacterial DNA, fungal DNA, viral RNA, or DNA or RNA found in bacterial or virus-infected cells. In an alternative embodiment, the nucleic acids to be detected can be synthetic or engineered DNA.

The method described herein can be used to provide the detection or characterization of specific nucleic acid sequences associated with infectious diseases, genetic disorders or cellular disorders such as cancers. It may also be used in forensic investigations and DNA typing. For purposes of this invention, genetic diseases include specific deletions or mutations in genomic DNA from any organism, such as sickle cell anemia, cystic fibrosis, alpha-thalassemia, beta-thalessemia, as well as other diseases apparent to a person of skill in the art. The molecules which may be amplified include any naturally occurring prokaryotic. For example, pathogenic or non-pathogenic bacteria, *Escherichia, Salmonella, Clostridium, Agrobacter, Staphylococcus, Streptomyces, Streptococcus, Rickettsiae, Chlamydia, Nycoplasma,* etc.), eukaryotic (e.g. protozoans and parasites, fungi, yeast, higher plants, lower and higher animals - including mammals and humans) or viral (e.g. herpes viruses, influenza virus, epstein-barr virus, hepatitis virus, polio virus, retroviruses, etc.) or viroid nucleic acid. The nucleic acid molecule can also be any nucleic acid molecule that has been or can be chemically synthesized. Thus, the nucleic acid sequence may or may not be found in nature.

Having now generally described the invention, the same will be more readily understood through reference to the following examples, which are provided by way of illustration, and are not intended to be limiting of the present invention

### EXAMPLES

### Example 1: Inhibition of Pyrococcus furiosus (Pfu) Polymerase Using dU-Containing Oligonucleotides.

In order to assess the inhibitory effect of dU-containing DNA on PCR using an archaebacterial DNA polymerase a dU-containing synthetic oligonucleotide was used in PCR reactions performed with Pfu DNA polymerase. Pfu polymerase was obtained from Stratagene Corp. (La Jolla, Ca). The PCR reactions were assembled using the buffer provided with the enzyme. Each 50 uL reaction PCR contained 1 unit of Pfu polymerase, 200uM dNTP's each(dA, dC, dT and dG), 2 mM Mg SO₄, 40 ng of human genomic DNA and 200 nM of each amplification primers. Different amplification reactions were set up with different amounts of dU-containing inhibitor ranging from 0 to 320 ng per reaction. The cycling conditions were: 1 minute at 95°C for initial denaturation followed by 35 cycles of 30 seconds at 94°C, 30 seconds at 55°C and 1 minute at 72°C. As can be seen in Figure 1 that there was a significant inhibition of PCR in the presence of 80 ng of inhibitor oligonucleotide, and complete inhibition using 160 ng and 320 ng when using primer set 20. On the other hand when using primer set 21 for PCR the inhibition was less effective and 320 ng of inhibitor primer was needed for complete inhibition of amplification.

### Example 2: Reversible Inhibition Of Pfu Polymerase by dU Inhibitor Oligo.

In order to test the reversibility of inhibition of Pfu Polymerase with dU-containing inhibitor a series of PCR reactions were set identical to the Example 1 with the following differences: The dU inhibitor used had the following sequence: 5'tgcgaauuccagccucuccagaaaggccc3', and was used at 0 nM, 100nM and 200 nM concentrations. It was shown that both concentrations were effective in inhibiting PCR effectively compared to the reaction that had no inhibitor. Identical reactions were also set up with 100 nM and 200 nM inhibitor and prior to start of the PCR reaction were incubated for 5 minute at 37°C in the presence of 1 unit of *E. coli* uracil DNA glycosylase (Invitrogen Corp). It was shown that addition of UDG effectively degraded the inhibitor and reversed the inhibition of PCR with the dU inhibitor. In addition to *E. coli* UDG we also tested a thermostable UDG from *Thermus thermophilus* ("Tth UDG") and incubated the reactions for 5 minute at 65°C prior to start of PCR cycling. It was shown that thermostable UDG was also capable of reversing the PCR inhibition by degrading the dU inhibitor at high temperatures prior to PCR and mediating hot start PCR reaction (Figure 2).

### Example 3: SYBR Green Real Time PCR.

Use of dU-containing inhibitors were also tested in real time PCR using SYBR green dye as the fluorescent detector. The reactions contained 0.2 mM of each dNTP, 300 nM of each primer in 1X PCR buffer (20 mM Tris pH 8.3, 50 mM KCl, 3 mM MgCl2), 300 nM of each primer, 1 unit of Taq polymerase or 1 unit of i-Taq DNA polymerase (BioRad Labs), 2% DMSO, 0.3X SYBR green dye. The reactions with AR3 inhibitor contained 25 pMoles of AR3 inhibitor. Some of the reaction contained 0.5 units of UDG from *Thermus thermophilus* ("Tth UDG") or UDG from *Thermatoga maritime* ("Tma UDG.") Using 10 ng of cDNA synthesized from HeLa total RNA and using *Homo sapiens* TRRAP protein ("TRRAP") mRNA we showed that these primers produced a nonspecific amplification product using Taq DNA polymerase alone without hot start. This nonspecific product was not present when antibody-mediated hot start enzyme was used. Using AR3 as the dU-containing inhibitor for Taq DNA polymerase was also effective in elimination of nonspecific product. However, if thermostable UDG was not used to degrade the inhibitor the sensitivity of the amplification was reduced, as evidenced by a delay in Ct in real time PCR. When thermostable Tth UDG was used the yield of amplification product was improved as well as the Ct value in real time PCR. Tma UDG was also effective in elimination of inhibitor and the nonspecific amplification product. These data are presented in Figures 3 a and b.

AR3 is a double stranded dU-containing oligonucleotide with the following sequences: AR3 Lower - auauaugggaguauauggauauaugggauaggg (3SP3), and AR3 Upper - cccuaucccauauauccauauacuccc (3SP3). TRRAP inhibitor is a double stranded dU-containing oligonucleotide with the following sequences: TRRAP-6365F - agtctttgggaggagccagt, and TRRAP-6464R - gcggataaggaagttcacca.

### Example 4: Effect of dU Inhibitors on PCR Sensitivity.

Although the nucleic acid inhibitors are effective in reducing or eliminating the nonspecific PCR fragments, another criteria for an effective methodology is that, the hot start mechanism should not reduce the sensitivity of PCR reactions and not interfere with other aspects of PCR. Three primer sets were used to test the sensitivity in the presence of dU inhibitors in real time PCR. As can be seen in Table 1 the presence of nucleic acid inhibitors during PCR reaction reduced the sensitivity (i.e., higher Ct values) for all three amplicons compared to Taq control. However, when these inhibitors were degraded with thermostable UDG in the beginning of PCR (and during PCR) the sensitivity was increased as evidenced by lower Ct values in real time PCR. These experiments were performed essentially the same as in example 3.

**Table 1. Effect of dU inhibitors on PCR Sensitivity.**

| **Enzyme / Inhibitor** | **Actin** | **Tubulin** | **3'ADAR** |
|---|---|---|---|
| **Taq** | **18.3** | **18.5** | **20.5** |
| | **18.8** | **18.2** | **20.1** |
| | **18.4** | **17.8** | **20.3** |
| **Taq+Abs** | **18** | **17.7** | **20.2** |
| | **18.1** | **18.2** | **20.4** |
| | **18** | **17.8** | **20.3** |
| **Taq+AR3** | **19.2** | **19.2** | **21.9** |
| | **19.2** | **19.7** | **22** |
| | **18.9** | **19.3** | **22.2** |
| **Taq+AR3+0.5uTTh** | **18.3** | **17.9** | **20.5** |
| | **18.2** | **17.7** | **20.6** |
| | **18.3** | **18.4** | **20.2** |

### Example 5:

Since the use of free dU inhibitor with DNA polymerase relies on affinity of DNA polymerase to DNA, it requires use of a relatively high concentration of DNA inhibitor for achieving a complete inhibition of activity. In order to reduce the concentration of inhibitor, use of dU-containing inhibitors was also tested by attaching the dU inhibitor to Taq DNA polymerase. The double stranded dU inhibitor AR4 was synthesized with an amine group at the 5' end of each oligonucleotide. The double stranded oligonucleotide was then mixed with Taq DNA polymerase in the presence of formaldehyde for attachment of amine oligonucleotide to the reactive amine groups on Taq DNA polymerase. Testing of DNA polymerase for activity showed that the polymerase activity was inhibited or reduced after attachment of dU inhibitor to DNA polymerase. A series of attachment conditions and concentrations of both formaldehyde and dU inhibitor were tested. It was found that a wide range of DNA concentrations could be used for attachment with a wide range of formaldehyde concentrations. Depending on the concentration of formaldehyde and DNA the length of the attachment reaction could be varied from few minutes to few hours. The amount of oligonucleotide, formaldehyde, time of attachment and temperature of attachment could be varied to produce different enzyme preparations with different properties in terms of polymerase activity and activation. The conditions for preparation of various enzymes are shown in Table 2. The following example demonstrates the results obtained with 3 different enzyme preparations.

**Table 2:Preparation of various hot-start enzymes and conditions for PCR.**

| **Formaldehyde Concentration** | **Amount of dU inhibitor** | **Reaction time** (**Min**) |
|---|---|---|
| 0.05% | 1pmole | 20min |
| 0.05% | 2pmole | 20min |
| 0.05% | 1.5pmole | 20min |
| 0.05% | 1pmole | 25min |
| 0.05% | 1.5pmole | 25min |
| 0.05% | 1pmole | 30min |

For functional analysis of DNA polymerase preparations a real-time PCR amplification was performed using primers designed for NDUFB7 (NDUFB7FWD 5'TGCGCATGAAGGAGTTTGAG 3'and NDUFB7REV 5'CAGATTTGCCGCCTTCTTCTC3'). The PCR reactions contained 3 ng of human genomic DNA as template, 0.2 units of Tma UDG, 1-1.5 units of various taq DNA polymerase preparations, 200 uM dNTP's and 300 nM of each primer in standard PCR buffer and SYBR green dye for detection. PCR were performed in a real-time DNA cycler(IQ cycler, BioRad Labs). The samples were incubated at 37 C for 20 minutes followed by a 10- min heating at 95C and were cycled between 95 C (10 seconds) and 60 C (45 seconds) for 45 cycles and data were collected. At the end of cycling melting curve analysis was performed for identification of genuine amplicons from the non-specific products. We have noticed that these primers have a tendency to produce a non-specific product when using Taq DNA polymerase. However, upon using a hot-start polymerase a desired and specific product can be produced using these primers which can easily be differentiated from non-specific product upon DNA melting analysis. PCR reactions were set up using these primers in a real-time PCR using SYBR green dye as the fluorescent detection method followed by DNA melting analysis in a BioRad IQ real-time DNA cycler. A master mix containing all compenent of the reaction was made except the DNA polymerase. Various DNA polymerases were then added to separate aliquots prior to start of PCR. For DNA polymerase preparations of the current invention with AR4 dU inhibitor attached to the polymerase, the DNA polymerase mix also contained 0.2 units of UDG from *Thermatoga maritima.*

The functional activities of various DNA polymerase preparations are shown in Figures 4-16. As can be seen, unmodified Taq DNA polymerase produces a non-specific DNA amplification product and fails to produce the desired amplification product for the primers used. When utilizing a hot start polymerase (i.e. antibody mediated hot-start, I-Taq DNA polymerase, BioRad labs) a specific and desired product is produced with good yield. The various DNA polymerase preparations of the current invention successfully amplified the correct product without producing significant amount of non-specific product. As can be seen from the figures the different concentrations of AR4 inhibitor and different time of attachment could be varied for optimizing the performance of the DNA polymerase preparation. In other experiments we have also used UDG from Thermus thermophilus with equal success.

### Figure 3. Sequence of AR4 double stranded inhibitor:

AR4 upper strand: 5' CCCUAUCCCAUAUAUCCAUCCACUCCC 3'
AR4 Lower strand: 5' AUAUAUGGGAGUGGAUGGAUAUAUGGGAUAGGG 3'

### Example 6: Pretreatment of polymerase prep with UDG:

Effect of UDG treatment on the Hot-start enzyme preparations of the invention were also examined in the following manner. We tested pretreatment of the enzyme preparation with UDG at room temperature and storage of the pretreated enzyme at various temperatures. The preparations were made as in the above example and were mixed with Tma or TTH UDG for 30 minutes at room temperature. The enzyme preps were tested in the functional real-time PCR assay as described above with NDUFB primers. It was found that the properties of the enzyme did not change as a result of pretreatment and the hotstart nature of the enzyme was not lost by pretreatment of preparation with UDG. Long term storage of the pretreated enzyme showed stable hot-start DNA polymerase. The pretreated preparations were stored at Room temp, 4C and at -20 C for longer than 48 hours and were found to be stable. These results shows that although one can add antibody against UDG to keep it from inactivating the inhibitor dU DNA, this methodology can also be practiced without antibodies. As part of these experiments it was also shown that a mastermix preparation of all required components (hot-start polymerase, UDG, dNTP's, SYBR green, Mg and buffer) for realtime PCR could be mixed together and stored at room temperature, 4C and -20 C. Such mastermixes were found to be stable and functioned in PCR similar to freshly made reagents.

**Table 3: Amount of AR4 double stranded**

| Preparation No. | inhibitor | Time of attachment |
|---|---|---|
| **1(** 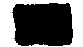 **)** | 1 pmole | 20min |
| **2(** 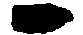 **)** | 2pmole | 20min |
| **3(** 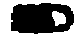 **)** | 1.5pmole | 20min |
| **Anti Taq Ab's (X)** | NA | NA |
| **Taq(▲)** | NA | NA |

### Example 7: Irreversible covalent attachment of inhibitor

Example 5 showed that a dU inhibitor that is covalently attached to a polymerase can be an effective way of inhibiting polymerase activity. The mechanism and chemistry used for attachment of polymerase is not limited and many different attachment methods can be used. To demonstrate this point we used DSG (disuccinimidyl glutarate, Pierce Biotechnology, Rockford, IL, Cat # 20593),a homo-bifunctional conjugation agent, for attaching an NH2-modified inhibitor nucleic acid to Taq DNA polymerase. The double stranded dU inhibitor AR4 was synthesized with an amine group at the 5' end of each oligonucleotide. The double stranded oligonucleotide was then mixed with Taq DNA polymerase in the presence of DSG for attachment of the amine oligonucleotide to the reactive amine groups on Taq DNA polymerase. Testing of DNA polymerase for activity showed that the polymerase activity was inhibited or reduced after attachment of dU inhibitor to DNA polymerase.

A series of attachment conditions and concentrations of both DSG and dU inhibitor were tested. It was found that a wide range of DNA concentrations could be used for attachment with a wide range of DSG concentrations. Depending on the concentration of DSG and DNA, the length of the attachment reaction could be varied from a few minutes to a few hours. The amount of oligonucleotide, DSG, time of attachment and temperature of attachment could be varied to produce different enzyme preparations with different properties in terms of polymerase activity and activation. The conditions for preparation of various enzymes are shown in Table 3. Figure 7 demonstrates the results obtained with 2 different enzyme preparations. It was also found that if the reactions were performed without AR4 inhibitor, Taq polymerase was modified and inactivated permanently resulting in non-functional protein (Figure 8).

For functional analysis of DNA polymerase preparations a real-time PCR amplification was performed using primers designed for NDUFB7 (NDUFB7FWD 5' TGCGCATGAAGGAGTTTGAG 3' and NDUFB7REV 5'CAGATTTGCCGCCTTCTTCTC3'). The PCR reactions contained 3 ng of human genomic DNA as template, 0.2 units of Tma UDG, 1-1.5 units of various taq DNA polymerase preparations, 200 uM dNTP's and 300 nM of each primer in standard PCR buffer and SYBR green dye for detection. PCR were performed in a real-time DNA cycler(IQ cycler, BioRad Labs). The samples were incubated at 37 C for 20 minutes followed by a 5-min heating at 95C and were cycled between 95 C (10 seconds) and 60 C (45 seconds) for 45 cycles and data were collected. At the end of cycling melting curve analysis was performed for identification of genuine amplicons from the non-specific products. We have noticed that these primers have a tendency to produce a non-specific product when using Taq DNA polymerase. However, upon using a hot-start polymerase a desired and specific product can be produced using these primers which can easily be differentiated from non-specific product upon DNA melting analysis. PCR reactions were set up using these primers in a real-time PCR using SYBR green dye as the fluorescent detection method followed by DNA melting analysis in a BioRad IQ real-time DNA cycler. A master mix was prepared containing all components of the reaction except the DNA polymerase. Various DNA polymerases were then added to separate aliquots prior to start of PCR. For DNA polymerase preparations of the current invention with AR4 dU inhibitor attached to the polymerase, the DNA polymerase mix also contained 0.2 units of UDG from Thermatoga maritima.

The functional activities of various DNA polymerase preparations are shown in Figure 7. As can be seen, unmodified Taq DNA polymerase produces a non-specific DNA amplification product and fails to produce the desired amplification product for the primers used. When utilizing a hot start polymerase (i.e. antibody mediated hot-start, I-Taq DNA polymerase, BioRad labs) a specific and desired product is produced with good yield. The various DNA polymerase preparations of the current invention successfully amplified the correct product without producing significant amount of non-specific product. As can be seen from the figures the different concentrations of AR4 inhibitor and different time of attachment could be varied for optimizing the performance of the DNA polymerase preparation. In other experiments UDG from Thermus thermophilus was used with equal success.

**Table 4: Preparation of various hot-start enzymes and conditions for preparation.**

| **Enzyme Prep.** | **NH2Ar4 /u Taq** | **DSG /u Taq** | **Time** |
|---|---|---|---|
| Prep 1 ( ○) | 5pmole | 10pmole | 10min |
| Prep 2 (■) | 5pmole | 20pmole | 10min |
| Anti-Taq Antibodies (X) | N/A | N/A | N/A |
| Taq (▲) | N/A | N/A | N/A |

### Example 8:

We also tested using another homobifunctional crosslinking agent, EGS, (ethylene glycol-bis succinimidylsuccinate available from Pierce Biotechnology, Rockford, IL, Cat # 20593). The double stranded dU inhibitor AR4 was synthesized with an amine group at the 5' end of each oligonucleotide. The double stranded oligonucleotide was then mixed with Taq DNA polymerase in the presence of EGS for attachment of amine oligonucleotide to the reactive amine groups on Taq DNA polymerase. Testing of DNA polymerase for activity showed that the polymerase activity was inhibited or reduced after attachment of dU inhibitor to DNA polymerase. A series of attachment conditions and concentrations of both EGS and dU inhibitor were tested. It was found that a wide range of DNA concentrations could be used for attachment with a wide range of EGS concentrations. Depending on the concentration of EGS and DNA, the length of the attachment reaction could be varied from few minutes to few hours. The amount of oligonucleotide, EGS, time of attachment and temperature of attachment could be varied to produce different enzyme preparations with different properties in terms of polymerase activity and activation. The conditions for preparation of various enzymes are shown in Figure legends (Figures 8-11).

Testing of the enzyme preparations were using the NDUFB primer set as described in example 7.

### Example 9:

The above examples tested use of homobifunctional conjugation agents as the method of attaching an AR4 inhibitor to a DNA polymerase molecule. These agents link the NH₂ group on the oligonucleotide to amine groups on the protein. We also tested heterobifunctional conjugation agents so that DNA inhibitor could be attached to different groups and/or sites on the DNA polymerase molecule. As an example we used carbodiimide which can link amine groups to COOH groups. Amine-modified AR4 DNA inhibitor was used and conjugation was attempted with 1-ethyl-3-(3-dimethylaminopropyl) carbodiimide hydrochloride.

A variety of conditions and concentrations of various components of the reaction were examined. The results are demonstrated in Figures 12 to 14. It was concluded that the mere presence of EDC in the amplification reaction with no time allowed for linking did not have any effect on the amplification and Taq polymerase exibibited the same activity in the presence or absence of EDC(see Fig. 12). When EDC was mixed with Taq in the absence of AR4 inhibitor and was allowed to react with Taq, it did not inhibit Taq activity substantially. Only when high amounts (40 pMoles) of EDC were used for extended period of reaction time (90 Min), was reduced Taq activity observed( Fig. 13 ).

AR4 inhibitor was conjugated to Taq DNA polymerase under a variety of conditions and parameters for reversible inhibition of polymerase were developed.

Figure 14 shows examples of hot-start Taq polymerase developed using this methodology. It was determined that the level of polymerase inhibition could be modulated and some preparations with excessive modification did not work well as they retained low or no polymerase activity.

Testing of the enzyme preparations were using the NDUFB primer set as described in example 7.

### Example 10: Development of monoclonal antibodies against Tth Uracil DNA glycosylase:

In certain applications of the present invention it would be desirable to incubate all reagents at ambient temperatures for an extended period of time. Therefore it would be helpful to neutralize enzymatic activity of UDG at low temperatures so that DNA polymerase remains inactive at low temperatures. One way to accomplish this is by using specific monoclonal or polyclonal antibodies that react with UDG and reversibly inhibit UDG activity. Since antibodies are thermolabile, when the reaction temperature is elevated during 1^{st} cycle of PCR antibodies are denatured and release active UDG into the reaction, which in turn, activate the DNA polymerase.

Using standard published methodologies both polyclonal and monoclonal antibodies were isolated against Tth UDG. A subset of antibodies that specifically bound to UDG, were also capable of partially or completely inhibiting UDG activity at low temperatures and released UDG at higher temperatures. Figure 16 demonstrates use on one monoclonal antibody in real time PCR experiments. The details of the experiments are the same as described for example 7 except that the PCR reaction contained 100 ng of monoclonal antibody against Tth UDG. 0.1 units of Tth UDG, and 1.5 units of DSG/AR4-modified Taq DNA polymerase. The complete PCR reaction mix was then incubated at room temperature for 5 hours prior to start of PCR. It was found that Taq DNA polymerase remained inactive at room temperature and retained its hot start function. The monoclonal antibody used effectively neutralized the UDG activity at room temperature and released fully active UDG during the denaturation step of PCR.

## Claims

1. A method of restoring the polymerase activity of a reversibly inhibited DNA polymerase enzyme, comprising:
contacting said polymerase enzyme with a second enzyme,
wherein said second enzyme modifies a nucleic acid binding partner that is bound to said polymerase enzyme,
wherein the polymerase activity of said polymerase enzyme is substantially inhibited by said binding partner,
and wherein modification of said binding partner by said second enzyme restores polymerase activity of said polymerase enzyme.

2. The method according to claim 1, wherein said DNA polymerase is a thermostable DNA polymerase.

3. The method according to claim 1, wherein said binding partner is non-covalently bound to said polymerase.

4. The method according to claim 3, wherein said binding partner is a single stranded nucleic acid.

5. The method according to claim 3, wherein said binding partner is a double stranded nucleic acid.

6. The method according to claim 1, wherein said binding partner is covalently bound to said polymerase.

7. The method according to claim 3, wherein said nucleic acid comprises a plurality of deoxyuridine residues and wherein said second enzyme has DNA glycosylase activity.

8. The method according to claim 6, wherein said second enzyme has DNA glycosylase activity.

9. The method according to claim 8, wherein said nucleic acid comprises at least one deoxyuridine residue, and wherein said second enzyme has UDG activity.

10. The method according to claim 9, wherein said nucleic acid is covalently bound to said polymerase by a covalent linkage that is heat-labile or hydrolytically labile during PCR temperature cycling.

11. The method according to claim 9, wherein said nucleic acid is covalently bound to said polymerase by a covalent linkage that is not heat-labile or hydrolytically labile during PCR temperature cycling.

12. The method according to claim 11, wherein said covalent linkage comprises an amide linkage, a urethane linkage, or a urea linkage.

13. The method according to claim 12, wherein said linkage comprises an amide linkage.

14. The method according to claim 6, wherein said nucleic acid comprises an RNA oligonucleotide, and said second enzyme has RNAse activity.

15. The method according to claim 14, wherein said nucleic acid is a double stranded nucleic acid comprising a DNA/RNA duplex and wherein said second enzyme has RNAseH activity.

16. The method according to claim 6, wherein said nucleic acid comprises a DNA/DNA duplex and wherein said second enzyme has restriction endonuclease activity that cleaves said duplex.

17. The method according to claim 16, wherein said second enzyme cuts at a 6-8 base pair recognition site.

18. The method of claim 1, wherein contacting said polymerase enzyme with a second enzyme, comprises:
contacting said polymerase enzyme with a second enzyme and an antibody that binds to said second enzyme and inhibits the activity of said enzyme at ambient temperature,
and wherein under PCR temperature cycling conditions the inhibition of activity of said second enzyme by said antibody is reduced or eliminated and modification of said binding partner by said second enzyme restores polymerase activity of said polymerase enzyme.

19. The method according to claim 18, wherein said DNA polymerase is a thermostable DNA polymerase.

20. The method according to claim 18, wherein said binding partner is non-covalently bound to said polymerase.

21. The method according to claim 18, wherein said binding partner is covalently bound to said polymerase.

22. The method according to claim 20, wherein said nucleic acid comprises a plurality of deoxyuridine residues and wherein said second enzyme has DNA glycosylase activity.

23. The method according to claim 21, wherein said second enzyme has DNA glycosylase activity.

24. The method according to claim 23, wherein said nucleic acid comprises at least one deoxyuridine residue, and wherein said second enzyme has UDG activity.

25. The method according to claim 21, wherein said nucleic acid comprises an RNA oligonucleotide, and said second enzyme has RNAse activity.

26. The method according to claim 25, wherein said nucleic acid is a double stranded nucleic acid comprising a DNA/RNA duplex and wherein said second enzyme has RNAseH activity.

27. The method according to any of claims 18-24, wherein said polymerase is an archaebactaerial polymerase.

28. The method according to claim 27, where said polymerase is Pfu.

29. The method according to any of claims 18-24, wherein said polymerase is a Eubacterial DNA polymerase.

30. The method according to claim 29, where said polymerase is Taq DNA polymerase.

31. The method according to any of claims 21-25, wherein said binding partner is a single stranded nucleic acid.

32. The method according to any of claims 21-25, wherein said binding partner is a double stranded nucleic acid.

33. The method according to claim 31 or 32, wherein said nucleic acid comprise at least one modified deoxyribonucleotide.

34. The method according to claim 33, wherein said nucleic acid comprises at least one deoxyuridine residue modifiable by an enzyme having UDG activity.

35. The method according to claim 21, wherein said nucleic acid comprises a DNA/DNA duplex and wherein said second enzyme has restriction endonuclease activity that cleaves said duplex.

36. The method according to claim 35, wherein said second enzyme cuts at a 6-8 base pair recognition site.

37. A reversibly inactivated DNA polymerase composition comprising a reversibly inactivated DNA polymerase, a nucleic acid binding partner bound to the DNA polymerase, and a second enzyme, wherein said DNA polymerase is active in the absence of said binding partner, and substantially inactive in the presence of said binding partner, and
wherein said binding partner is modifiable by a the second enzyme, whereby modification of said binding partner by said second enzyme restores polymerase activity to said polymerase enzyme.

38. The polymerase composition according to claim 37, where said polymerase is an archaebactaerial polymerase.

39. The polymerase composition according to claim 37, wherein said polymerase is Pfu.

40. The polymerase composition according to claim 37, wherein said polymerase is a Eubacterial DNA polymerase.

41. The polymerase composition according to claim 37, wherein said polymerase is Taq DNA polymerase.

42. The polymerase composition according to claim 37, wherein said DNA polymerase is a thermostable DNA polymerase.

43. The polymerase composition according to claim 37, wherein said binding partner is non-covalently bound to said polymerase.

44. The polymerase composition according to claim 43, wherein said nucleic acid comprises at least one modified deoxyribonucleotide.

45. The polymerase composition according to claim 43, wherein said nucleic acid comprises at least one deoxyuridine residue modifiable by an enzyme having UDG activity.

46. The polymerase composition according to claim 37, wherein said binding partner is covalently bound to said polymerase.

47. The polymerase composition according to claim 46, wherein said nucleic acid comprises at least one deoxyuridine residue modifiable by an enzyme having UDG activity.

48. The polymerase composition according to claim 47, wherein said nucleic acid is covalently bound to said polymerase by a covalent linkage that is not heat-labile or hydrolytically labile during PCR temperature cycling.

49. The polymerase composition according to claim 48, wherein said covalent linkage comprises an amide linkage, a urethane linkage, or a urea linkage.

50. The polymerase composition according to claim 49, wherein said linkage comprises an amide linkage.

51. The polymerase composition according to claim 46, wherein said nucleic acid comprises an RNA oligonucleotide.

52. The polymerase composition according to claim 46, wherein said nucleic acid is a double stranded nucleic acid comprising a DNA/RNA duplex that is recognized by an enzyme having RNAseH activity.

53. The polymerase composition according to claim 46, wherein said nucleic acid comprises a DNA/DNA duplex containing a recognition site recognized by a restriction endonuclease.

54. The polymerase composition according to claim 53, wherein said recognition site is 6-8 bases long.

## Patentansprüche

1. Verfahren zum Wiederherstellen der Polymerase-Aktivität eines reversibel inhibierten DNA-Polymerase-Enzyms, umfassend:
Kontaktieren des Polymerase-Enzyms mit einem zweiten Enzym,
wobei das zweite Enzym einen Nukleinsäurebindungspartner modifiziert, der an das Polymerase-Enzym gebunden ist,
wobei die Polymerase-Aktivität des Polymerase-Enzyms durch den Bindungspartner im Wesentlichen inhibiert ist,
und wobei Modifikation des Bindungspartners durch das zweite Enzym Polymerase-Aktivität des Polymerase-Enzyms wiederherstellt.

2. Verfahren nach Anspruch 1, wobei die DNA-Polymerase eine thermostabile DNA-Polymerase ist.

3. Verfahren nach Anspruch 1, wobei der Bindungspartner nicht-kovalent an die Polymerase gebunden ist.

4. Verfahren nach Anspruch 3, wobei der Bindungspartner eine einzelsträngige Nukleinsäure ist.

5. Verfahren nach Anspruch 3, wobei der Bindungspartner eine doppelsträngige Nukleinsäure ist.

6. Verfahren nach Anspruch 1, wobei der Bindungspartner kovalent an die Polymerase gebunden ist.

7. Verfahren nach Anspruch 3, wobei die Nukleinsäure eine Vielzahl von Desoxyuridin-Resten umfasst und wobei das zweite Enzym DNA-Glycosylase-Aktivität aufweist.

8. Verfahren nach Anspruch 6, wobei das zweite Enzym DNA-Glycosylase-Aktivität aufweist.

9. Verfahren nach Anspruch 8, wobei die Nukleinsäure wenigstens einen Desoxyuridin-Rest aufweist und wobei das zweite Enzym UDG-Aktivität aufweist.

10. Verfahren nach Anspruch 9, wobei die Nukleinsäure kovalent an die Polymerase durch eine kovalente Bindung gebunden ist, die während PCR-Temperaturzyklisierung Hitze-labil oder hydrolytisch labil ist.

11. Verfahren nach Anspruch 9, wobei die Nukleinsäure kovalent an die Polymerase durch eine kovalente Bindung gebunden ist, die während PCR-Temperaturzyklisierung nicht Hitze-labil oder hydrolytisch labil ist.

12. Verfahren nach Anspruch 11, wobei die kovalente Bindung eine Amidbindung, eine Urethanbindung oder eine Harnstoffbindung umfasst.

13. Verfahren nach Anspruch 12, wobei die Bindung eine Amidbindung umfasst.

14. Verfahren nach Anspruch 6, wobei die Nukleinsäure ein RNA-Oligonukleotid umfasst und das zweite Enzym RNAse-Aktivität aufweist.

15. Verfahren nach Anspruch 14, wobei die Nukleinsäure eine doppelsträngige Nukleinsäure ist, die einen DNA/RNA-Duplex umfasst und wobei das zweite Enzym RNAseH-Aktivität aufweist.

16. Verfahren nach Anspruch 6, wobei die Nukleinsäure einen DNA/DNA-Duplex umfasst und wobei das zweite Enzym Restriktionsendonuklease-Aktivität aufweist, die den Duplex spaltet.

17. Verfahren nach Anspruch 16, wobei das zweite Enzym eine Erkennungsstelle aus 6-8 Basen schneidet.

18. Verfahren nach Anspruch 1, wobei Kontaktieren des Polymerase-Enzyms mit einem zweiten Enzym umfasst:
Kontaktieren des Polymerase-Enzyms mit einem zweiten Enzym und einem Antikörper, der an das zweite Enzym bindet und die Aktivität des Enzyms bei Umgebungstemperatur inhibiert,
und wobei unter PCR-Temperaturzyklisierungsbedingungen die Inhibierung von Aktivität des zweiten Enzyms durch den Antikörper verringert oder beseitigt ist und Modifikation des Bindungspartners durch das zweite Enzym Polymerase-Aktivität des Polymerase-Enzyms wiederherstellt.

19. Verfahren nach Anspruch 18, wobei die DNA-Polymerase eine thermostabile DNA-Polymerase ist.

20. Verfahren nach Anspruch 18, wobei der Bindungspartner nicht-kovalent an die Polymerase gebunden ist.

21. Verfahren nach Anspruch 18, wobei der Bindungspartner kovalent an die Polymerase gebunden ist.

22. Verfahren nach Anspruch 20, wobei die Nukleinsäure eine Vielzahl von Desoxyuridin-Resten umfasst und wobei das zweite Enzym DNA-Glycosylase-Aktivität aufweist.

23. Verfahren nach Anspruch 21, wobei das zweite Enzym DNA-Glycosylase-Aktivität aufweist.

24. Verfahren nach Anspruch 23, wobei die Nukleinsäure wenigstens einen Desoxyuridin-Rest umfasst und wobei das zweite Enzym UDG-Aktivität aufweist.

25. Verfahren nach Anspruch 21, wobei die Nukleinsäure ein RNA-Oligonukleotid umfasst und das zweite Enzym RNAse-Aktivität aufweist.

26. Verfahren nach Anspruch 25, wobei die Nukleinsäure eine doppelsträngige Nukleinsäure ist, die einen DNA/RNA-Duplex umfasst, und wobei das zweite Enzym RNAseH-Aktivität aufweist.

27. Verfahren nach einem der Ansprüche 18-24, wobei die Polymerase von Archaebacterium ist.

28. Verfahren nach Anspruch 27, wobei die Polymerase Pfu ist.

29. Verfahren nach einem der Ansprüche 18-24, wobei die Polymerase eine DNA-Polymerase von Eubacterium ist.

30. Verfahren nach Anspruch 29, wobei die Polymerase Taq DNA-Polymerase ist.

31. Verfahren nach einem der Ansprüche 21-25, wobei der Bindungspartner eine einzelsträngige Nukleinsäure ist.

32. Verfahren nach einem der Ansprüche 21-25, wobei der Bindungspartner eine doppelsträngige Nukleinsäure ist.

33. Verfahren nach Anspruch 31 oder 32, wobei die Nukleinsäure wenigstens ein modifiziertes Desoxyribonukleotid umfasst.

34. Verfahren nach Anspruch 33, wobei die Nukleinsäure wenigstens einen Desoxyuridin-Rest umfasst, der durch ein Enzym mit UDG-Aktivität modifizierbar ist.

35. Verfahren nach Anspruch 21, wobei die Nukleinsäure einen DNA-DNA-Duplex umfasst und wobei das zweite Enzym Restriktionsendonuklease-Aktivität aufweist, die den Duplex spaltet.

36. Verfahren nach Anspruch 35, wobei das zweite Enzym eine Basenpaarerkennungsstelle aus 6-8 Basen schneidet.

37. Zusammensestzung einer reversibel inaktivierten DNA-Polymerase umfassend eine reversibel inaktivierte DNA-Polymerase, einen Nukleinsäurebindungspartner, der an die DNA-Polymerase gebunden ist, und ein zweites Enzym, wobei die DNA-Polymerase in Abwesenheit des Bindungspartners aktiv ist und in Gegenwart des Bindungspartners im Wesentlichen inaktiv ist, und
wobei der Bindungspartner durch das zweite Enzym modifizierbar ist, wobei Modifikation des Bindungspartners durch das zweite Enzym Polymerase-Aktivität des Polymerase-Enzyms wiederherstellt.

38. Polymerasenzusammensetzung nach Anspruch 37, wobei die Polymerase eine Polymerase von Archaebacterium ist.

39. Polymerasenzusammensetzung nach Anspruch 37, wobei die Polymerase Pfu ist.

40. Polymerasenzusammensetzung nach Anspruch 37, wobei die Polymerase eine DNA-Polymerase von Eubacterium ist.

41. Polymerasenzusammensetzung nach Anspruch 37, wobei die Polymerase Taq DNA-Polymerase ist.

42. Polymerasenzusammensetzung nach Anspruch 37, wobei die DNA-Polymerase eine thermostabile DNA-Polymerase ist.

43. Polymerasenzusammensetzung nach Anspruch 37, wobei der Bindungspartner nicht-kovalent an die Polymerase gebunden ist.

44. Polymerasenzusammensetzung nach Anspruch 43, wobei die Nukleinsäure wenigstens ein modifiziertes Desoxyribonukleotid umfasst.

45. Polymerasenzusammensetzung nach Anspruch 43, wobei die Nukleinsäure wenigstens einen Desoxyuridin-Rest umfasst, der durch ein Enzym mit UDG-Aktivität modifizierbar ist.

46. Polymerasenzusammensetzung nach Anspruch 37, wobei der Bindungspartner kovalent an die Polymerase gebunden ist.

47. Polymerasenzusammensetzung nach Anspruch 46, wobei die Nukleinsäure wenigstens einen Desoxyuridin-Rest umfasst, der durch ein Enzym mit UDG-Aktivität modifizierbar ist.

48. Polymerasenzusammensetzung nach Anspruch 47, wobei die Nukleinsäure kovalent an die Polymerase durch eine kovalente Bindung gebunden ist, die während PCR-Temperaturzyklisierung nicht Hitze-labil oder hydrolytisch labil ist.

49. Polymerasenzusammensetzung nach Anspruch 48, wobei die kovalente Bindung eine Amidbindung, eine Urethanbindung oder eine Harnstoffbindung umfasst.

50. Polymerasenzusammensetzung nach Anspruch 49, wobei die Bindung eine Amidbindung umfasst.

51. Polymerasenzusammensetzung nach Anspruch 46, wobei die Nukleinsäure ein RNA-Oligonukleotid umfasst.

52. Polymerasenzusammensetzung nach Anspruch 46, wobei die Nukleinsäure eine doppelsträngige Nukleinsäure ist umfassend einen DNA/RNA-Duplex, der durch ein Enzym mit RNAseH-Aktivität erkannt wird.

53. Polymerasenzusammensetzung nach Anspruch 46, wobei die Nukleinsäure einen DNA/DNA-Duplex umfasst enthaltend eine Erkennungsstelle, die durch eine Restriktionsendonuklease erkannt wird.

54. Polymerasenzusammensetzung nach Anspruch 53, wobei die Erkennungsstelle 6-8 Basen lang ist.

## Revendications

1. Procédé de restitution de l'activité de polymérase d'une enzyme ADN polymérase inhibée de manière réversible, comprenant les étapes consistant à :
mettre en contact ladite enzyme polymérase avec une deuxième enzyme,
dans laquelle ladite deuxième enzyme modifie un partenaire de liaison à l'acide nucléique, qui est lié à ladite enzyme polymérase,
dans laquelle l'activité de polymérase de ladite enzyme polymérase est substantiellement inhibée par ledit partenaire de liaison,
et dans laquelle une modification dudit partenaire de liaison par ladite deuxième enzyme restitue l'activité de polymérase de ladite enzyme polymérase.

2. Procédé selon la revendication 1, dans laquelle ladite ADN polymérase est une ADN polymérase thermostable.

3. Procédé selon la revendication 1, dans laquelle ledit partenaire de liaison est lié, de manière non covalente, à ladite polymérase.

4. Procédé selon la revendication 3, dans laquelle ledit partenaire de liaison est un acide nucléique en simple brin.

5. Procédé selon la revendication 3, dans laquelle ledit partenaire de liaison est un acide nucléique en double brin.

6. Procédé selon la revendication 1, dans laquelle ledit partenaire de liaison est lié, de manière covalente, à ladite polymérase.

7. Procédé selon la revendication 3, dans laquelle ledit acide nucléique comprend une pluralité de résidus de désoxyuridine et dans laquelle ladite deuxième enzyme a une activité d'ADN glycosylase.

8. Procédé selon la revendication 6, dans laquelle ladite deuxième enzyme a une activité d'ADN glycosylase.

9. Procédé selon la revendication 8, dans laquelle ledit acide nucléique comprend au moins un résidu de désoxyuridine, et dans laquelle ladite deuxième enzyme a une activité d'UDG.

10. Procédé selon la revendication 9, dans laquelle ledit acide nucléique est lié, de manière covalente, à ladite polymérase par une liaison covalente qui est thermolabile ou instable à l'hydrolyse lors de la cyclisation thermique dans une ACP.

11. Procédé selon la revendication 9, dans laquelle ledit acide nucléique est lié, de manière covalente, à ladite polymérase par une liaison covalente qui n'est pas thermolabile ou n'est pas hydrolytiquement instable lors de la cyclisation thermique dans une ACP.

12. Procédé selon la revendication 11, dans laquelle ladite liaison covalente comprend une liaison amide, une liaison uréthane ou une liaison urée.

13. Procédé selon la revendication 12, dans laquelle ladite liaison comprend une liaison amide.

14. Procédé selon la revendication 6, dans laquelle ledit acide nucléique comprend un oligonucléotide d'ARN et ladite deuxième enzyme a une activité de RNAse.

15. Procédé selon la revendication 14, dans laquelle ledit acide nucléique est un acide nucléique en double brin comprenant un duplexe d'ADN/ARN et dans laquelle ladite deuxième enzyme a une activité de RNAseH.

16. Procédé selon la revendication 6, dans laquelle ledit acide nucléique comprend un duplexe d'ADN/ADN et dans laquelle ladite deuxième enzyme a une activité d'endonucléase de restriction qui coupe ledit duplexe.

17. Procédé selon la revendication 16, dans laquelle ladite deuxième enzyme coupe au niveau d'un site de reconnaissance de 6 à 8 paires de bases.

18. Procédé selon la revendication 1, dans laquelle la mise en contact de ladite enzyme polymérase avec une deuxième enzyme comprend :
la mise en contact de ladite enzyme polymérase avec une deuxième enzyme et un anticorps, qui se lie à ladite deuxième enzyme et qui inhibe l'activité de ladite enzyme à température ambiante,
et dans laquelle, dans des conditions de cyclisation thermique dans une ACP,
l'inhibition de l'activité de ladite deuxième enzyme par ledit anticorps est réduite ou supprimée et la modification dudit partenaire de liaison par ladite deuxième enzyme restitue l'activité de polymérase de ladite enzyme polymérase.

19. Procédé selon la revendication 18, dans laquelle ladite ADN polymérase est une ADN polymérase thermostable.

20. Procédé selon la revendication 18, dans laquelle ledit partenaire de liaison est lié, de manière non covalente, à ladite polymérase.

21. Procédé selon la revendication 18, dans laquelle ledit partenaire de liaison est lié, de manière covalente, à ladite polymérase.

22. Procédé selon la revendication 20, dans laquelle ledit acide nucléique comprend une pluralité de résidus de désoxyuridine et dans laquelle ladite deuxième enzyme a une activité d'ADN glycosylase.

23. Procédé selon la revendication 21, dans laquelle ladite deuxième enzyme a une activité d'ADN glycosylase.

24. Procédé selon la revendication 23, dans laquelle ledit acide nucléique comprend au moins un résidu de désoxyuridine, et dans laquelle ladite deuxième enzyme a une activité d'UDG.

25. Procédé selon la revendication 21, dans laquelle ledit acide nucléique comprend un oligonucléotide d'ARN et ladite deuxième enzyme a une activité de RNAse.

26. Procédé selon la revendication 25, dans laquelle ledit acide nucléique est un acide nucléique en double brin comprenant un duplexe d'ADN/ARN et dans laquelle ladite deuxième enzyme a une activité de RNAseH.

27. Procédé selon l'une quelconque des revendications 18 à 24, dans laquelle ladite polymérase est une polymérase archaebactérienne.

28. Procédé selon la revendication 27, dans laquelle ladite polymérase est une Pfu.

29. Procédé selon l'une quelconque des revendications 18 à 24, dans laquelle ladite polymérase est une ADN polymérase eubactérienne.

30. Procédé selon la revendication 29, dans laquelle ladite polymérase est une ADN polymérase Taq.

31. Procédé selon l'une quelconque des revendications 21 à 25, dans laquelle ledit partenaire de liaison est un acide nucléique en simple brin.

32. Procédé selon l'une quelconque des revendications 21 à 25, dans laquelle ledit partenaire de liaison est un acide nucléique en double brin.

33. Procédé selon la revendication 31 ou la 32, dans laquelle ledit acide nucléique comprend au moins un désoxyribonucléotide modifié.

34. Procédé selon la revendication 33, dans laquelle ledit acide nucléique comprend au moins un résidu de désoxyuridine, modifiable par une enzyme ayant une activité d'UDG.

35. Procédé selon la revendication 21, dans laquelle ledit acide nucléique comprend un duplexe d'ADN/ADN et dans laquelle ladite deuxième enzyme a une activité d'endonucléase de restriction qui coupe ledit duplexe.

36. Procédé selon la revendication 35, dans laquelle ladite deuxième enzyme coupe au niveau d'un site de reconnaissance de 6 à 8 paires de bases.

37. Composition d'ADN polymérase réversiblement inactivée comprenant une ADN polymérase réversiblement inactivée, un partenaire de liaison à l'acide nucléique étant lié à l'ADN polymérase, ainsi qu'une deuxième enzyme, dans laquelle ladite ADN polymérase est active en l'absence dudit partenaire de liaison et est substantiellement inactive en présence dudit partenaire de liaison, et dans laquelle ledit partenaire de liaison est modifiable par la deuxième enzyme, moyennant quoi une modification dudit partenaire de liaison par ladite deuxième enzyme restitue l'activité de polymérase à ladite enzyme polymérase.

38. Composition de polymérase selon la revendication 37, dans laquelle ladite polymérase est une polymérase archaebactérienne.

39. Composition de polymérase selon la revendication 37, dans laquelle ladite polymérase est une Pfu.

40. Composition de polymérase selon la revendication 37, dans laquelle ladite polymérase est une ADN polymérase eubactérienne.

41. Composition de polymérase selon la revendication 37, dans laquelle ladite polymérase est une ADN polymérase Taq.

42. Composition de polymérase selon la revendication 37, dans laquelle ladite ADN polymérase est une ADN polymérase thermostable.

43. Composition de polymérase selon la revendication 37, dans laquelle ledit partenaire de liaison est lié, de manière non covalente, à ladite polymérase.

44. Composition de polymérase selon la revendication 43, dans laquelle ledit acide nucléique comprend au moins un désoxyribonucléotide modifié.

45. Composition de polymérase selon la revendication 43, dans laquelle ledit acide nucléique comprend au moins un résidu de désoxyuridine modifiable par une enzyme ayant une activité d'UDG.

46. Composition de polymérase selon la revendication 37, dans laquelle ledit partenaire de liaison est lié, de manière covalente, à ladite polymérase.

47. Composition de polymérase selon la revendication 46, dans laquelle ledit acide nucléique comprend au moins un résidu de désoxyuridine modifiable par une enzyme ayant une activité d'UDG.

48. Composition de polymérase selon la revendication 47, dans laquelle ledit acide nucléique est lié, de manière covalente, à ladite polymérase par une liaison covalente qui n'est pas thermolabile ou n'est pas hydrolytiquement instable lors de la cyclisation thermique dans une ACP.

49. Composition de polymérase selon la revendication 48, dans laquelle ladite liaison covalente comprend une liaison amide, une liaison uréthane ou une liaison urée.

50. Composition de polymérase selon la revendication 49, dans laquelle ladite liaison comprend une liaison amide.

51. Composition de polymérase selon la revendication 46, dans laquelle ledit acide nucléique comprend un oligonucléotide d'ARN.

52. Composition de polymérase selon la revendication 46, dans laquelle ledit acide nucléique est un acide nucléique en double brin comprenant un duplexe d'ADN/ARN, qui est reconnu par une enzyme ayant une activité de RNAseH.

53. Composition de polymérase selon la revendication 46, dans laquelle ledit acide nucléique comprend un duplexe d'ADN/ADN contenant un site de reconnaissance reconnu par une endonucléase de restriction.

54. Composition de polymérase selon la revendication 53, dans laquelle ledit site de reconnaissance a une longueur de 6 à 8 bases.
